# EUROPEAN PATENT APPLICATION

(11) **EP 1 900 377 A1**
(43) Date of publication of application: **19.03.2008**
(21) Application number: 06120540.7
(22) Date of filing: 12.09.2006
(51) Int. Cl.: A61K 39/395, A61K 39/00, A61K 31/7088, A61K 48/00, A61K 51/10, A61K 47/48, A61P 35/00, C07K 16/28, C07K 16/00, C12N 15/11, G01N 33/53, G01N 33/68, C07K 19/00, C07K 14/12, C07K 14/47, C12N 15/12

(54) **Inhibitors of GPR87 for cancer therapy**

(71) Applicant: Boehringer Ingelheim International GmbH, 55216 Ingelheim am Rhein (DE)
(72) Inventor: Glatt, Sebastian, 1030 Wien (AT); Sommergruber, Wolfgang, 3002 Purkersdorf (AT); Haslinger, Christian, 1080 Wien (AT); Stratowa, Christian, 1010 Wien (AT); Garin-Chesa, Pilar, 1230 Wien (AT)
(74) Representative: Hammann, Heinz

(57) **Abstract**

Use of GPR87 for the generation of GPR87 inhibitors. GPR87 inhibitors, e.g. anti-GPR87 antibodies and small molecules, are useful as drugs in the treatment of cancer. Methods for identifying small molecule GPR87 inhibitors employ cells transfected with GPR87 or GPR87-G-alpha fusion proteins.

## Description

### Technical field of the invention

The present invention relates to the therapy and diagnosis of cancer.

### Background of the invention

The search for tumor targets, i.e. molecules which are expressed preferentially or exclusively in cancerous tissue, is essential for the development of novel strategies for treatment and diagnosis of cancer. The recent advantages in microarray/oligonucleotide array technology have enabled to study the complex distribution of transcribed transcripts all over different tissues and cell types. These developments in connection with the statistically relevant availability of clinical tissue samples from cancer patients and the corresponding non-cancerous tissue samples allow the identification of tumor specific transcribed mRNA transcripts. Usually the level of mRNA available per single cell correlates with the level of functional translated protein per cell. For this reason, tumor specific transcripts code for essential biological functions for the analyzed tumor cells. Therefore, identifying and characterizing biological functions that substantially contribute to tumorigenesis, is indispensable for developing new therapies to intervene in the progress of tumor formation and tumor establishment.

The detailed analysis of tumor specific transcripts, the essential biological functions of the corresponding proteins and their annotation to a certain tumor type not only allows the design of highly specific tumortherapies. Compared to general therapeutic concepts such as anti-mitotic approaches, the molecularly defined therapy bears a lower risk for off-target effects. It also carries the potential to avoid ineffective tumor-therapies for patients who have developed tumors that do not exhibit elevated levels of the respective transcript, and will therefore not benefit from inhibiting the therapy-targeted biological function. In contrast, molecule-based strategies allow a more sophisticated and effective combination of diagnosis and therapy.

One strategy for targeting tumor cells is based on identifying proteins that are localized on the cell surface. These proteins can be targeted directly at their extra-cellular domains. Therefore, in drug development, solving the problem of membrane permeability is dispensable for compounds that target cell surface proteins and their biological function, respectively.

Genes encoding GTP-binding protein coupled receptors (GPCRs) represent approximately 3-5% of all human genes and therefore are one of the largest gene families known within the human genome. The family of GPCRs exhibits sufficient variation in structural targets to fulfill the criteria of specificity and drugibility. It is therefore not surprising that estimated 30% of the current pharmaceutical compounds target GPCRs (Hopkins and Groom, 2002). Particularly, the essential contribution of members of this protein family (including G-protein-coupled receptor kinases) to tumorigenesis has already been demonstrated by a huge number of publications e.g. (Li et al., 2005; Metaye et al., 2005). The different biological processes that involve GPCR-regulated signaling are as diverse as the specific stimuli, which trigger these signaling cascades. The to date identified ligands, which bind and activate those downstream cascades of GPCRs, are manifold, ranging from biogenic amids, nucleotides, peptides, amino acids, odorants, lipids, ions or even photons. The signals generated by the binding of the specific ligand are transmitted into the cell through the activation of heterotrimeric GTP-binding proteins (G-proteins), protein complexes consisting of alpha, beta, and gamma subunits (Hepler and Gilman, 1992). In the human genome, 16 alpha, 5 beta, and 12 gamma subunits are annotated. Although one G-protein can promiscuously bind to different GPCRs and transmit the generated signals, the G-protein/GPCR interaction is highly specific. The structural changes induced by the binding of an adequate ligand facilitate the exchange of GDP by GTP on the G-alpha subunit of the heterotrimeric G-protein. Because of these changes in the affinity for a nucleotide, the heterotrimeric G-protein complex dissociates into the alpha and beta/gamma sub-complexes, which sequentially interact with their specific substrates in the cell. Activated G-alpha proteins (GTP-bound) can regulate intracellular calcium, adenylyl cyclases, phospholipases, Rho-family members, protein kinase C, ion channels and phosphodiesterases. Among these different signaling pathways, regulated by activated GPCRs almost all are known to be altered in neoplastic diseases (Marinissen and Gutkind, 2001). A variety of potent mitogens such as thrombin, bombesin, and bradykinin, to name but a few, are known, which stimulate cell proliferation by acting on their cognate GPCRs in many different cell types (Gutkind, 1998). One of the first GPCRs identified to provide a link between cellular transformation and GPCRs was the mas oncogene (Young et al., 1986); mutations in the mas oncogene that result in agonist-independent activation were identified. Besides the agonist-independent activation of GPCRs by acquired genetic mutations, the ectopic expression *per se* of wild-type 5-HT2C and muscarinic acetylcholine M1, M3 and M5 receptors was shown to be able transform murine fibroblasts (NIH 3T3) in an agonist-dependent fashion, providing evidence that wild-type GPCRs can be tumorigenic when exposed to an excess of locally produced or circulating agonists. In addition, mutated GPCRs might be rendered transforming even in an agonist-independent fashion, as shown, for example, for α1B-adrenoceptors (Allen et al., 1991). For an overview on mutant G-protein-coupled receptors and their involvement in human diseases see (Schoneberg et al., 2004). For example, naturally occurring constitutively activating mutations were found in some differentiated thyroid carcinomas (Parma et al., 1993). Although activating mutations are infrequent in GPCRs, these receptors can often contribute to tumorigenesis when persistently stimulated with agonists released from tumors. For example, bombesin, gastrin-releasing peptide (GRP), neuromedin B (NMB), bradykinin, cholecystokinin (CCK), galanin, neurotensin, and vasopressin are secreted by small-cell lung carcinoma cells, which also express their cognate GPCRs and are thus activated in an autocrine or paracrine fashion (Heasley, 2001).

The importance of purines (e.g. adenosine, ADP, ATP) and pyrimidines (e.g. UDP, UTP, UDP-Glucose) as signaling molecules was suggested by Burnstock and Kennedy in 1985, proposing the existence of highly specific receptors for these nucleotides - the P2 receptor family (Burnstock and Kennedy, 1985). This receptor family is structurally divided into two subfamilies - ionotropic ligand-dependent ion channels (P2X) and metabotropic GPCRs (P2Y) (Fredholm et al., 1997). After the recognition of purines and pyrimidines as GPCR agonists intensive work was put forward to identify the nucleotide-regulated signaling pathways. P2Y receptor activation was shown to mediate proliferation through the activation of the MAPK pathway in C6 glioma cells (Tu et al., 2000), reduce TNF-alpha induced apoptosis in astrocytes (Kim et al., 2003), stimulate DNA synthesis and the accumulation of cAMP in swiss3T3 cells, and can activate early mitogenic responses, such as increase of intracellular free calcium and accumulation of inositol phosphates (Gonzalez et al., 1990).

### Summary of the invention

It was an object of the invention to identify GPCRs which play an essential role in tumorigenesis and to provide inhibitors of such novel cancer-relevant GPCRs for cancer therapy.

To solve the problem underlying the invention, first, a transcriptional profiling analysis was done that was based on expression profiles on Affymetrix Gene Chips HG-U133A and B of the GeneLogic database (Shen-Ong et al., 2003). Cluster analysis (normal and tumor tissues versus transcriptional profile) revealed GPR87 as a GPCR with the most tumor-specific transcription profile.

Little is known about human GPR87 (hGPR87, protein sequence NP_076404), an "orphan GPCR" for which no agonists have yet been identified which stimulate downstream cascades.The hGPR87 protein sequence is highly homologous to P2Y14 (41% amino acid identity, GENESTREAM network server, IGH, Montpellier, FRANCE), which was demonstrated to be the specific receptor for UDP-glucose. Furthermore, the motifs known to be important for nucleotide binding in TM6 and TM7 in P2Y14 are also conserved in hGPR87 (Abbracchio et al., 2003).
The phylogenetic analysis by Joost and Methner (Joost and Methner, 2002), who included 277 human GPCRs in their study, revealed that human GPR87 has significant sequence homology with the P2Y subfamily. Based on this study, the inventors performed an in-depth homology analysis of the P2Y subfamily protein sequences in order to identify those members with the highest homology with hGPR87. The closest relatives of hGPR87 are P2Y12 (NP_795345), P2Y13 (NP_795713), and P2Y14 (NP_055694).

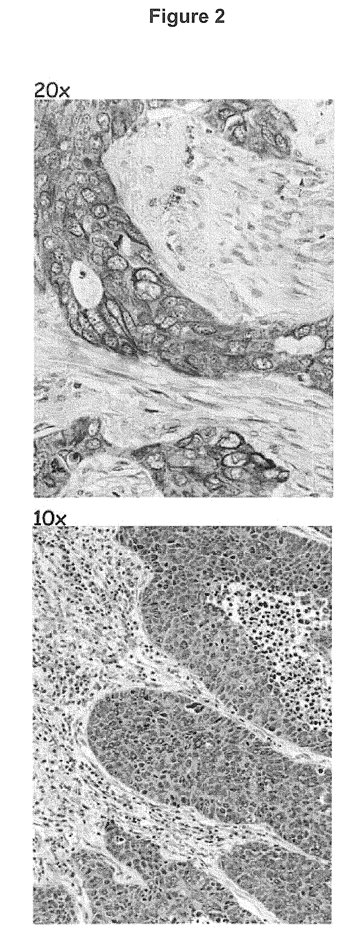

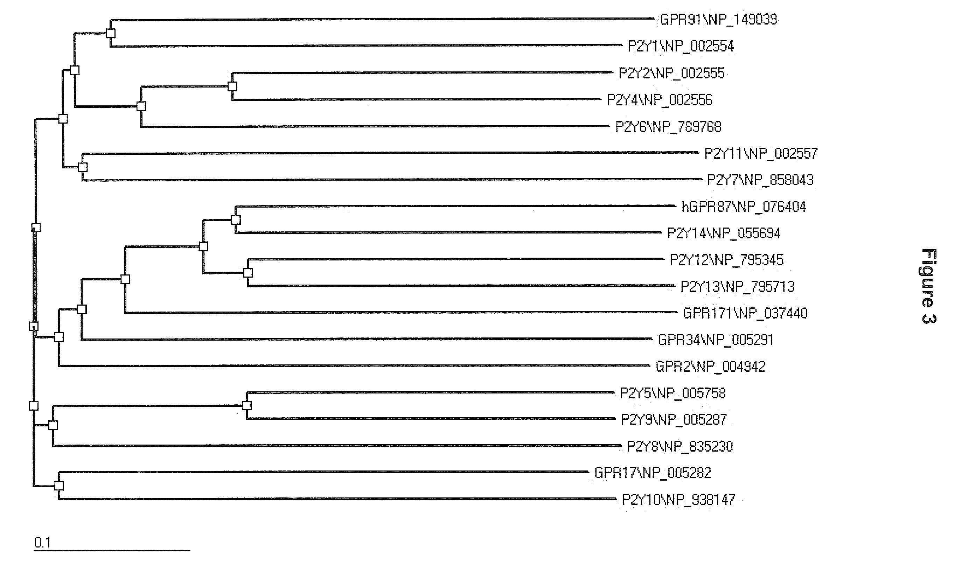

Figure 3 shows the homology tree of the P2Y subfamily. The nearest homologues of hGPR87 are P2Y12, P2Y13, and P2Y14. (To generate the neighbor-joining-tree of the P2Y receptor family including hGPR87, Clustalw software package was used. The sequences were all obtained from NCBI database.)
The nucleotide sequence of human GPR87 (hGPR87) corresponds to the sequence of Genbank Accession No. NM_23915. Relating to this data base entry, the start codon is defined as being at position 362, the encoded protein thus starting with MGFNL (aa positions 1 to 5 of SEQ ID NO:2). The proposed N-terminal extracellular domain ranges from aa 1 to aa 46 of SEQ ID NO:2. The seven proposed transmembrane domains (TM) of human GPR87 are spanning from aa 47 to aa 68 (TM1), aa 79 to aa 99 (TM2), aa 119 to aa 137 (TM3), aa 160 to aa 180 (TM4), aa 206 to aa 229 (TM5), aa 253 to aa 271 (TM6), and aa 298 to aa 317 (TM7) of SEQ ID NO:2. The three intracellular loops (ILs) of human GPR87 are spanning from aa 69 to aa 78 (IL1), aa 138 to aa 159 (IL2), and aa 230 to aa 252 (IL3) of SEQ ID NO:2. The three extracellular loops (ELs) of human GPR87 are spanning from aa 100 to aa 118 (EL1), aa 181 to aa 205 (EL2), and aa 272 to aa 297 (EL2) of SEQ ID NO:2. The intracellular C-terminus of human GPR87 is located between aa 318 to aa 358 of SEQ ID NO:2. The DRY motif, typical for GPCRs is located between aa 138 and aa 140 of SEQ ID NO:2. Another highly conserved GPCR motif of human GPR87 is located between aa 310 and a 314 of of SEQ ID NO:2. The intracellular C-terminus of human GPR87 includes a proposed PDZ binding site, which consists of aa 356, aa 357, and aa 358 of SEQ ID NO:2.

Most G-protein coupled receptors have single conserved cysteine residues in each of the first two extracellular loops which form disulfide bonds that are believed to stabilize functional protein structure. These conserved structural motifs are represented by cysteine 114 and cysteine 192 of SEQ ID NO:2, which are located in the extracellular loop one and two, respectively.

In a general aspect, the present invention relates human GPR87 as a target for therapeutic intervention, in particular in the treatment of cancer.

Thus, in a first aspect, the present invention relates to the use of the hGPR87 polypeptide (SEQ ID NO:2) or of hGPR87 cDNA (SEQ ID NO:1) or of GPR87 polypeptide or polynucleotide molecules that have sequences sufficiently identical to these sequences and variants and fragments thereof for the generation of inhibitors of GPR87.

The term "sufficiently identical" refers to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common functional activity. Variant nucleic acid molecules and polypeptides substantially homologous to the nucleotide and amino acid sequences set forth in the sequence listings are encompassed to be used according to the present invention.

In particular, the use of a polynucleotide that hybridizes with a polynucleotide of the sequence shown in SEQ ID NO:1 or under stringent conditions, and a polypeptide encoded by such polynucleotide, is encompassed.

By "stringent hybridization conditions" as used herein is meant overnight incubation at 42°C in a solution comprising: 50% formamide, 5x SSC (1X SSC = 150 mM NaCl, 15mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C, or equivalent conditions.

In the following, the term "GPR87" encompasses the above-defined GPR87 molecules with functional activity common to naturally occurring GPR87.

In a specific aspect, the present invention relates to an inhibitor of human GPR87 function for use as an anti-tumor agent; the GPR87 function comprising, as first found by the present inventors, the generation of signals for survival, proliferation and growth for tumor cells. Furthermore, GPR87 is involved in late stage tumorigenesis such as the onset of metastases and lymph node infiltration and may also be involved in migration.

In the following, an inhibitor of GPR87 function is termed as "GPR87 inhibitor".

In the following, if not otherwise defined, "GPR87" stands for human GPR87 (=hGPR87).

In a first aspect, the GPR87 inhibitor is an anti-GPR87 antibody. The invention also relates to the use of an anti-GPR87 antibody in the therapy and diagnosis of cancer.

In another aspect, the GPR87 inhibitor is a small molecule. The invention also relates to the identification and characterization of such small molecule GPR87 inhibitors and their use in cancer therapy.

In another aspect of the invention, the GPR87 inhibitor is a GPR87 antisense molecule.

In a further aspect, the invention relates to the production of GPR87 polypeptides.

In still another aspect, the invention relates to the use GPR87 for the generation and characterization of an anti-hGPR87 antibody.

In another aspect, the invention relates to GPR87-G-alpha fusion polypeptides and their production.

The GPR87-G-alpha fusion polypeptides of the invention include, but are not limited to, fusion polypeptides of GPR87 with GNAQ (Homo sapiens guanine nucleotide binding protein alpha q. DNA sequence AF493896; protein sequence AAM12610); GNASL (Homo sapiens guanine nucleotide binding protein alpha s long. DNA sequence AF493897; protein sequence AAM12611); GNASS (Homo sapiens guanine nucleotide binding protein alpha s short. DNA sequence AF493898; protein sequence AAM12612) and GNA15 (Homo sapiens guanine nucleotide binding protein alpha 15. DNA sequence AF493904; protein sequence AAM12618). These G-alpha proteins are reviewed in (Seifert et al., 1999b).

In another aspect, the invention relates to methods for using GPR87 polypeptides and/or GPR87-G-alpha fusion polypeptides as well as the polynucleotide molecules encoding them. Usage of polynucleotides includes transfection into cells of interest and the generation of stable cell lines derived therefrom that express the corresponding polypeptides.

In another aspect of the invention, GPR87 expressing cells are used in assay methods that allow to determine a compound's effect on GPR87 activation. Such assays may be employed as screening methods to identify compounds that modulate GPR87 function, in particular antagonists (usually also referred to as a small molecule inhibitors). In the case of antagonists, the compounds bind to the receptor, but do not elicit a second messenger response, such that the activity of the receptor is prevented; in the case of agonists an activation is observed.

Preferably, assays are done in the high-throughput screening (HTS) format.

In the following, assay methods that are useful to determine a compound's effect on GPR87 activation, including screening methods, are termed "GPR87 assays" or "GPR87 assay methods". Apart from being useful in screening methods that identify a GPR87 small molecule inhibitor out of a large number of compounds, e.g. by screening compound libraries, GPR87 assays are also useful in drug development, e.g. for profiling a compound that has been identified as a hit in an antecedent primary or secondary screening assay.

A preferred GPR87 assay method uses a cell that expresses an GPR87-G-alpha fusion polypeptide and is capable of generating a detectable signal that is dependent on the binding of the ligand to GPR87 and thus receptor activation. As described in more detail below, signals include e.g. alterations in the cAMP levels and changes in the intracellular calcium concentrations or in the intra- and extracellular pH value, signals resulting from activation of the MAPK pathway via the phospholipase C/ PKC/RAF1/MEK cascade (e.g. phosphorylation of MEK or MAPK), binding/competition of fluorescence-labeled ligands, internalization via ß-arrestin, as well as signals generated by reporter systems.

### Detailed description of the invention

In the experiments of the invention, it could be shown that an antibody against the N-terminus of human GPR87 inhibits proliferation of cells of a human head and neck carcinoma cell line that expresses human GPR87 endogenously. From the obtained results (PARP cleavage), it can be suggested that the reduced proliferation of the cells is accompanied by the activation of caspase-dependent apoptosis. The anti-proliferative as well as the apoptotic effects can be rescued by a blocking peptide corresponding to the N-terminal amino acid sequence of human GPR87. Taken together, the inventors have provided the first evidence that a GPR87 inhibitor (in the form of an anti-hGPR87 antibody) can inhibit the growth of human cancer cells.

Based on these findings, the present invention relates to anti-hGPR87 antibody molecules and their use in cancer therapy.

In a further aspect, the present invention relates to a pharmaceutical composition containing, as the active ingredient, an anti-hGPR87 antibody molecule, for the treatment of cancer.

In yet another aspect, the invention relates to the use of hGPR87 or a preparation containing hGPR87 for the generation of antibody molecules that specifically bind to the extracellular N-terminus of human GPR87 expressed by a tumor cell, thereby antagonizing the activation of human GPR87 and inhibiting the proliferation of tumor cells. (In the following, such antibody molecule is referred to as "anti-hGPR87 antibody").

The DNA sequence encoding full-length hGPR87 mRNA is shown in SEQ ID NO:1, the amino acid sequence encoded by this sequence is shown in SEQ ID NO:2.

Herein, "anti-GPR87 antibody molecule" shall mean that the antibody molecule has specific binding affinity to an epitope, which is present in the extracellular N-terminus of human GPR87 polypeptide corresponding to amino acids 1 - 46 of SEQ ID: NO 2, or a portion thereof. In view of using the antibody in animal studies, it may be advantageous if the antibody is reactive with both human GPR87 and GPR87 of the respective animal used in the studies, e.g. mouse in pharmacological studies or Cynomolgus in toxicology studies.

"Specific binding", as defined herein, means binding to human GPR87, or a fragment thereof, respectively, and does not exclude crossreactivity with GPR87 from another mammalian species. This definition also does not exclude that the antibody molecule may be able to bind, in addition to GPR87, to one or more other proteins of interest.

The term "anti-GPR87 antibody molecule" encompasses anti-GPR87 antibodies, anti-GPR87 antibody fragments, anti-GPR87 antibody-like molecules and conjugates with any of the above mentioned antibody molecules. Antibodies include, but are not limited to, polyclonal, monoclonal, chimerized monoclonal, humanized monoclonal, fully human monoclonal, and fully human polyclonal, bispecific, and heteroconjugate antibodies. Thus, the term "antibody" shall encompass complete immunoglobulins as they are produced by lymphocytes and for example present in blood sera, monoclonal antibodies secreted by hybridoma cell lines, polypeptides produced by recombinant expression in host cells, which have the binding specificity of immunoglobulins or monoclonal antibodies, and molecules which have been derived from such immunoglobulins, monoclonal antibodies, or polypeptides by further processing while retaining their binding specificity.

In particular, the term "antibody molecule" includes complete immunoglobulins comprising two heavy chains and two light chains like murine, rat, hamster and rabbit origin, chimeric, humanized or fully human antibodies (Breitling and Duebel, 1999; Shin and Morrison, 1989; Gussow and Seemann, 1991; Winter et al., 1994; EP 0 239 400; EP 0 519 596; WO 90/07861; EP 0 368 684; EP 0 438 310; WO 92/07075; WO 92/22653; EP 0 680 040; EP 0 451 216), as well as fragments of such immunoglobulins like Fab, Fab', or F(ab)₂ fragments (Kreitman et al., 1993), single chain antibodies (Johnson and Bird, 1991), and polypeptides that contain variable regions (e.g. small modular immunopharmaceutics, SMIPs) or individual complementary determining regions (CDRs) as present in immunoglobulin, and the like.

To serve as an immunogen for obtaining antibodies or antibody-like molecules of the invention, the entire GPR87 polypeptide or fragments thereof may be used, expressed as a recombinant protein, or chemically synthesized peptides containing sequences of human GPR87 and in particular the extracellular N-terminus, optionally bound to a carrier molecule, to elicit lymphocytes that produce antibodies binding to the extracellular domain of human GPR87. Alternatively, immunization can be done with a recombinantly produced GPR87 extracellular N-terminus or portions thereof, or with a fusion protein including the GPR87 extracellular N-terminus sequence or a portion thereof, e.g. a fusion protein consisting of a portion of GPR87 and a fusion partner that provides a tag for purification and/or secretion, e.g. a Heme Agglutinin (HA) tag or a portion of CD8 that includes both an affinity tag and a leader sequence. Alternatively to using the N-terminus, peptides from other extracellular domains of h GPR87 may be used, e.g. peptides from the extracellular loops EL1 or EL2, as defined above.

An antigenic GPR87-derived peptide useful as an immunogen comprises at least 8, preferably 10 to 30 amino acid residues and encompasses an epitope of human GPR87 within the extracellular N-terminus such that an antibody raised against the peptide forms a specific immune complex with human GPR87. By way of example, in the experiments of the present invention, hGPR87 peptides encompassing the first 46 amino acids, or the last 41 amino acids, respectively,of SEQ ID NO:2 were successfully used to block the affinity of generated antibodies against GPR87.

Alternatively to using purified human GPR87 proteins or peptides for immunization, DNA-based immunization can be used as strategy for the production of monoclonal antibodies, bypassing the step of antigen purification (Wolff et al., 1990; Barry et al., 1994), optionally in the form of electroporation in conjunction with intramuscular immunization (Aihara and Miyazaki, 1998; Widera et al., 2000).

Alternatively, cell lines expressing human GPR87 (endogenously or upon transfection with human GPR87, or parts thereof that are linked to the cell membrane) can be used as an immunogen to generate anti-GPR87 antibodies.

Alternatively to using human GPR87, a GPR87 orthologue from another species (e.g. murine, bovine or monkey GPR87) may be used as an immunogen, as defined above, as long as the obtained antibody (molecule) crossreacts with human GPR87 and exhibits the desired therapeutic effects in humans.

In one embodiment, the anti-GPR87 antibody is a polyclonal antibody, which can be prepared by immunizing a suitable animal (e.g. rabbit, goat, mouse, rat, hamster, donkey, cow or another mammal) with a GPR87 immunogen. The antibody titer in the immunized subject can be monitored by standard techniques, such as with an enzyme linked immunosorbent assay (ELISA) or FACS analysis. Polyclonal anti-GPR87 antibodies are particularly useful for conducting experiments that show an anti-tumor effect, e.g. a direct effect by anti-proliferative or pro-apoptotic activity or an indirect effect (in the case of binding to human GPR87 localized at the cell surface) by antibody-dependent cellular cytotoxicity (ADCC; Eccles, 2001) or complement-dependent cytotoxicity (CDC).

In a further embodiment, the anti-GPR87 antibody is a monoclonal antibody.

To obtain monoclonal anti-GPR87 antibodies, the lymphocytes obtained from the immunized host animal or a human donor with GPR87 reactive lymphocytes or, alternatively, lymphocytes obtained by immunization *in vitro,* as described by (Tsai et al., 1998) are fused with an immortalized cell line to form hybridoma cells. Immortalized cell lines are usually transformed mammalian cells, particularly myeloma cells, e.g. rat, rabbit, mouse or human myeloma cell lines. The hybridoma cells are then cultured in a suitable culture medium that inhibits growth or survival of the unfused, immortalized cells. The culture medium is then tested for the presence of anti-GPR87 monoclonal antibodies according to established methods.

The obtained clones may be subcloned and grown by standard methods. The monoclonal antibodies may be isolated or purified from the culture medium by conventional immunoglobulin purification procedures. Methods for producing monoclonal antibodies were also described by e.g. (Harlow, 1988)

By way of example, if monoclonal antibodies are generated by immunizing mice with GPR87 and standard hybridoma technology, as described above, they can be characterized and further developed as follows: The antibodies are tested for specific binding to GPR87 protein and peptide fragments thereof and to human GPR87 expressed on the cell surface. These antibodies are useful for analyzing cells and tissues for the expression of GPR87, for example by FACS analysis, immunofluorescence (IF) or immunohistochemistry (IHC). The antibodies are analyzed for their functional potential like GPR87-mediated cell proliferation or the induction of apoptosis.

The present invention provides, for the first time, a molecule that neutralizes the activity of GPR87, in particular, anti-GPR87 antibodies with anti-proliferative and pro-apoptotic activities for use in therapy.

The obtained antibodies can be further characterized by first determining the nucleotide sequences encoding for the respective antibodies according to standard methods, as described e.g. by Nayak et al., 1998. Then the epitopes on GPR87 that are recognized by the antibodies can be determined. Cross reactivity with GPR87 of other species and homologue human proteins, like other members of the GPCR family -in particular members of the P2Y purinoceptor subfamily- can be analyzed. Antibodies can be tested for activities with respect to receptor mediated internalization, induction of effector functions like ADCC and CDC and anti-tumor effects *in vivo.* Effector functions can be enhanced by modifying the Fc-part of the antibody, in particular by protein engineering (Shields et al., 2001; Lazar et al., 2006) or by altering glycosylation of the antibody, e.g. by lowering the fucose content of the glycostructure (Niwa et al., 2005). Based on the sequence of these antibodies, in particular the sequence of an antibody that shows the desired antagonistic effects, chimeric and/or humanized antibodies, can be generated as described below. The thus obtained antibodies, which are also subject of the present invention, may be used as an anti-cancer therapeutic to treat human patients.

Another method suitable for obtaining monoclonal anti-GPR87 antibodies is described in US 5,958,765 and Brams et al., 1998; it combines *in vitro* priming of human spleen cells and antigen boosting in SCID mice. This method provides for very high human antibody titers which are predominantly of the IgG isotype.

Alternatively, monoclonal anti-GPR87 antibodies may be obtained by the so-called "SLAM (Selected Lymphocyte Antibody Method) technology", described in US 5,627,052. According to this method, without prior immortalization a B cell is identified and isolated that expresses the desired GPR87 antibody from a population of B cells by a specific selection system and the antibody is synthesized after amino acid or DNA sequencing.

Alternatively, monoclonal antibodies can be selected from combinatorial libraries, by technologies like phage display, bacterial display, yeast display, ribosomal display and others as described below.

Monoclonal anti-GPR87 antibodies may also be obtained by recombinant DNA methods, such as those described in US 4,816,567. DNA encoding the monoclonal anti-GPR87 antibodies can be isolated and sequenced by routinely used procedures, e.g. by using oligonucleotide probes that bind to sequences encoding the heavy and light chains of murine antibodies. As a source of such DNA, hybridomas or clones from combinatorial libraries may be used. The DNA isolated is then inserted into expression vectors, which are transfected into host cells, e.g. COS cells or CHO cells to obtain the monoclonal antibodies in the recombinant host cells. Alternatively, expression of recombinant antibodies in transgenic animals, in non-mammalian systems, like *Aspergillus* and other microorganisms, and in plants are possible and have been described.

In a further aspect, the antibody molecule is an anti-GPR87 antibody-fragment that has an antigen binding region. To obtain antibody fragments, e.g. Fab fragments, digestion can be accomplished by means of routine techniques, e.g. using papain or pepsin. Examples of papain digestion are described in WO 94/29348 and US 4,342,566. Papain digestion of antibodies typically produces two identical antigen binding fragments, so-called Fab fragments, each with a single antigen binding site, and a residual Fc fragment. Pepsin treatment yields an F(ab')₂ fragment that has two antigen combining sites and is still capable of cross-linking the antigen.

The Fab fragments obtained by digestion of the antibody also contain the constant domains of the light chain and the first constant domain (CH₁) of the heavy chain. Fab' fragments differ from Fab fragments in that they contain additional residues at the carboxy terminus of the heavy chain CH₁ domain including one or more cysteines from the antibody hinge region.

Fab'-SH is the designation herein for Fab' in which the cysteine residue(s) of the constant domains bear a free thiol group. F(ab')₂ antibody fragments originally were produced as pairs of Fab' fragments which have hinge cysteines between them. Antibody fragments can also be generated by molecular biology methods producing the respective coding DNA fragments. Antigen-binding antibody fragments, including single-chain antibodies and linear antibodies as described in Zapata et al. (1995), may comprise the variable region(s) alone or in combination with the entirety or a portion of the following: constant domain of the light chain, CH1, hinge region, CH2, and CH3 domains. Also included in the invention are antigen-binding fragments also comprising any combination of variable region(s) with a constant domain region of a light chain,VH1, CH1, hinge region, CH2, and CH3 domains.

The antibody molecules may contain all or only a portion of the constant region as long as they exhibit specific binding to the relevant portion of the GPR87 antigen. The choice of the type and length of the constant region depends on whether effector functions like complement fixation or antibody dependent cellular toxicity are desired, and on the desired pharmacological properties of the antibody protein. The antibody molecule will typically be a tetramer consisting of two light chain/heavy chain pairs, but may also be dimeric, i.e. consisting of a light chain/heavy chain pair, e.g. a Fab or Fv fragment, or it may be a monomeric single chain antibody (scFv).

In another embodiment, the anti-GPR87 antibody molecule may be a so-called antibody-like molecule, which is a polypeptide containing short sequences or fragments of immunoglobulins. In particular, these are polypeptides containing one or more antigen binding regions that are identical or similar to a complementarity determining region (CDR) of an immunoglobulin. Such molecules can be single domain antibodies, e.g. so-called "nanobodies", which are micro-scaffolds comprising a CDR2 or CDR3 polypeptide sequence interconnecting fragments of the adjacent framework polypeptide sequences, which are arranged to form two anti-parallel strands (described e.g. in WO 03/050531 and by Revets et al., 2005). Other exmples for antibody-like molecules are immunoglobulin super family antibodies (IgSF; Srinivasan and Roeske, 2005), camelized antibodies or other CDR containing or CDR grafted molecules or "Domain Antibody" (dAb). dABs are functional binding units of antibodies, corresponding to the variable regions of either the heavy (VH) or light (VL) chains of human antibodies. Domain Antibodies have a molecular weight of approximately 13 kDa, or less than one-tenth the size of a full antibody. A series of large and highly functional libraries of fully human VH and VL dAbs has been developed. dABs are also available for "dual targeting", i.e. dAbs that bind, in addition to GPR87, to a second target in one molecule. dAb libraries, selection and screening methods, dAb formats for dual targeting and for conferring extended serum half life are described in e.g. US 6,696,245, WO 04/058821, WO 04/003019 and WO 03/002609.

In general antibody fragments and antibody-like molecules are well expressed in bacterial, yeast, and mammalian cell systems. Alternatively, the anti-GPR87 antibody-like molecule may be a so-called "SMIP" ("Small Modular Immunopharmaceutical"). This molecule employs a single polypeptide chain as its binding domain Fv, which is linked to single-chain hinge and effector domains devoid of the constant domain CH1 (WO 02/056910). The molecules can be prepared as monomers or dimers, but they do not assume the dimer-of-dimers structure of traditional antibodies.

The anti-GPR87 antibody molecule may be monovalent, divalent (diabody), trivalent (triabody), or tetravalent (tetrabody), meaning that it may bind to one or more epitopes of GPR87 and/or, in addition to GPR87, to the epitope(s) of one or more other proteins of interest. Methods for preparing such antibody molecules are well known in the art (e.g. reviewed by Hudson and Souriau, 2001).

In a further aspect, the anti-GPR87 antibody molecule is a chimeric antibody. The construction and production of chimeric rodent/human antibodies is well known in the art (Boulianne et al., 1984). The variable regions of the non-human antibody, e.g. a murine monoclonal antibody, are typically linked to at least a portion of the constant regions of a human immunoglobulin. Human constant region DNA sequences can be isolated in accordance with well-known procedures from a variety of human cells, preferably from immortalized B cells (see Kabat et al., 1991; *supra,* and WO 87/02671).

In a further aspect, the anti-GPR87 antibody is a humanized antibody. Humanized forms of non-human (e.g. murine) antibodies are chimeric forms of immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (from the recipient antibody) in which residues from a complementarity determining region (CDR) of the recipient antibody are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues.

"Complementarity determining regions" of an antibody are understood to be those amino acid sequences involved in specific antigen binding according to Kabat et al., 1991, in connection with Chothia and Lesk, 1987.

To obtain a humanized antibody, the antigen binding sites, which are formed by the CDRs of the heavy chain and CDRs of the light chain, are excised from the DNA of cells secreting rodent (murine) monoclonal antibody and grafted into the DNA coding for the framework of the human antibody. Since only the antigen-binding site CDRs, rather than the entire variable domain of the rodent antibody, e.g. mouse antibody, are transplanted, the resulting humanized antibody (second generation antibody) is less immunogenic than a first generation chimeric antibody.

"Complementarity determining regions" (CDRs) of a monoclonal antibody, as mentioned above, are understood to be those amino acid sequences involved in specific antigen binding according to Kabat et al., 1991, in connection with Chothia and Lesk, 1987. From the sequences of the variable regions as contained in the monoclonal antibodies of the invention, the CDR sequence can be routinely determined by searching the Kabat sequence database for sequence features.

Appropriate framework residues of the CDR-grafted antibody may be reverted to murine residues to improve binding affinity. As described above, from methods pertinent to the art, the experts knows how to obtain the CDRs from the sequence of a given monoclonal antibody, to choose and obtain appropriate human immunoglobulin genes, to graft the CDRs into these genes, to modify selected framework residues, to express the CDR-grafted antibody in appropriate host cells, e.g. Chinese hamster ovary (CHO) cells, and to test the resulting recombinant antibodies for binding affinity and specificity (see e.g. literature references above).

Alternatively to CDR grafting, antibodies can be humanized by other technologies whereby single amino acids are exchanged in the framework of the variable regions of the antibody to convert them to more human-like sequences, like for example the so-called "resurfacing" technology whereas the murine frameworks are left unchanged with the exception of surface-exposed residues (US 5,639,641).

Nucleic acid molecules coding for the light chain and the heavy chain may be synthesized chemically and enzymatically (PCR amplification) by standard methods. First, suitable oligonucleotides can be synthesized with methods known in the art (e.g. Gait, 1984), which can be used to produce a synthetic gene. Methods to generate synthetic genes from oligonucleotides are known in the art (e.g. Stemmer et al., 1995; Ye et al., 1992; Hayden and Mandecki, 1988; Frank et al., 1987).

The nucleic acid molecules encoding the antibody heavy and light chains may be cloned into an expression vector (either both chains in one vector molecule, or each chain into a separate vector molecule), which then is introduced into a host cell. Expression vectors suitable for immunoglobulin expression in prokaryotic or eukaryotic host cells and methods of introduction of vectors into host cells are well-known in the art, e.g. in transgenic animals, in non-mammalian system, like *Aspergillus* and other microorganisms, and in plants. In general, the immunoglobulin gene therein is in functional connection with a suitable promoter, like for example a human cytomegalovirus (CMV) promoter, hamster ubiquitin promoter (WO 97/15664), or a simian virus SV40 promoter located upstream of the lg gene. For termination of transcription, a suitable termination/polyadenylation site like that of the bovine growth hormone or SV40 may be employed. Furthermore, an enhancer sequence may be included, like the CMV or SV40 enhancer. Usually, the expression vector furthermore contains selection marker genes like the dihydrofolate reductase (DHFR), glutamine synthetase, adenosine deaminase, adenylate deaminase genes, or the neomycin, bleomycin, or puromycin resistance genes. A variety of expression vectors are commercially available from companies such as Stratagene, La Jolla, CA; Invitrogen, Carlsbad, CA; Promega, Madison, Wl or BD Biosciences Clontech, Palo Alto, CA. For example, expression vectors pAD-CMV1 (NCBI GenBank Accession No. A32111) or pAD-CMV19 (NCBI GenBank Accession No. A32110) may be used for expression. The host cell preferably is a mammalian host cell, e.g. a COS, CHO, or BHK cell, more preferably a chinese hamster ovary (CHO) cell, e.g. a CHO-DUKX (Urlaub and Chasin, 1980), CHO-DG44 (Urlaub et al., 1983), or CHO-K1 (ATCC CCL-61) cell. The host cell then is cultured in a suitable culture medium under conditions where the antibody is produced, and the antibody is then isolated from the culture according to standard procedures. Procedures for production of antibodies from recombinant DNA in host cells and respective expression vectors are well-known in the art (see e.g. WO 94/11523, WO 97/9351, EP 0 481 790, EP 0 669 986).

In a further aspect, the anti-GPR87 antibody may be a camelized antibody; methods for producing such antibodies are described by Tanha et al., 2001.

In a preferred embodiment, the anti-GPR87 antibody is a fully human antibody. Fully human antibodies may be obtained according to known methods, e.g. by phage display methods (Aujame et al., 1997; McCafferty et al. 1990; US 5,885,793; US 5,969,108; WO 97/08320, US 6,300,064; US 6,248,516, US 6,291,158). In brief, a monoclonal anti-GPR87 antibody can be obtained by screening a recombinant combinatorial immunoglobulin library (e.g. an antibody phage display library) against GPR87 (or a preparation containing the antigen, e.g. cells expressing GPR87) to isolate molecules that bind the GPR87 protein.

Completely human antibodies that recognize a selected epitope can also be generated using a technique referred to as "guided selection", which is based on the display of repertoires of human antibody fragments on filamentous phages and selection by binding of the phage to the antigen. In this approach, a selected non-human monoclonal antibody, e.g. a murine antibody, is used to guide the selection of a completely human antibody recognizing the same epitope. This technology was described e.g. by Jespers et al., 1994, and Figini et al., 1998, who generated a human antibody against a cell surface antigen that is overexpressed in many human ovarian carcinomas. The guiding template was provided by the light chain of a mouse monoclonal antibody which was paired with repertoires of human heavy chains displayed on phages. The guiding template may also be the mouse monoclonal antibody heavy chain to select repertoires of human light chain displayed on phages.

Another method for obtaining fully human antibodies utilizes surface display on prokaryotic cells like *Escherichia coli* and fluorescence-activated cell sorting (FACS), as described by Daugherty et al., 1998 and in WO 98/49286.

Fully human antibodies may also be produced according to the yeast display technology, as described by Colby et al., 2004, and in US patents 6,699,658, 6,696,251, 6,423,538 and 6,300,065.

Another suitable method to obtain the antibodies of the invention is the ribosome display method, a cell-free system for the *in vitro* selection of proteins and peptides from large libraries, as described by He and Taussig, 2002.

Completely human antibodies can also be produced using transgenic mice that are incapable of expressing endogenous immunoglobulin heavy and light chain genes, but can express human heavy and light chain genes, e.g. as described by Lonberg and Huszar, 1995; US 5,625,126; US 5,633,425; US 5,569,825; US 5,661,016; US 5,545,806; EP 0 438 474; EP 0 463 151; EP 0 546 073.

More specifically, a fully human anti-GPR87 antibody can for example be obtained by immunizing a transgenic mouse having a human immunoglobulin repertoire with the purified extracellular domain of human GPR87 (or fragments thereof) or fusion proteins containing the relevant antigenic portion of GPR87 or cells expressing GPR87 or relevant antigenic portions, or with DNA containing the respective GPR87 sequence or portions thereof. Said transgenic mice may be crossed with GPR87 knock out mice to enhance the possibilities to generate antibodies that are cross-reactive with murine GPR87. Antibody-secreting hybridomas are generated by standard methods and the variable light and heavy chains are cloned and sequenced. Antibodies can be produced either by hybridomas or by cloning the variable regions into expression vectors carrying human constant region immunoglobulin sequences for heavy and light chains and expressed in mammalian cells.

Alternatively, fully human monoclonal antibodies can be obtained in mice according to a method that comprises *in vitro* priming of human spleen cells and antigen boosting in immunodeficient mice, like SCID mice, as described in US 5,958,765.

An anti-GPR87 antibody may be generated such that it recognizes an epitope within the extracellular domain of GPR87 that is identical or overlaps with the epitope recognized by an antibody that has shown the desired inhibitory effect in preliminary experiments. Overlapping epitopes can for example be determined by competitive binding. Competitive binding may be determined in ELISA using plates coated with the GPR87 protein or GPR87 peptides or with GPR87 positive cells (Cell ELISA) and measuring binding of the biotinylated antibody in presence of a competitor antibody. In the presence of a competing antibody or antibody-derived fragment the binding of biotinylated antibody is reduced in the case that the antibodies recognize a shared epitope. Alternatively, Biacore analysis may be employed for testing antibodies with regard to competitive binding. Alternatively, tryptic digestion of a GPR87 molecule bound to the antibody of interest and analysis of the remaining bound GPR87 fragment may be used to identify the binding epitope. Binding of the antibody to peptides or protein fragments of GPR87 can narrow down the epitope recognized by the antibody.

Peptides or protein fragments that contain the thus identified epitope, or DNA molecules encoding such peptides/fragments, respectively, may be used for immunization to obtain antibodies reactive with the same epitope as the antibody of interest.

The anti-GPR87 antibody may be from any class, preferred anti-GPR87 antibodies belong to the IgG class, preferably the anti-GPR87 antibody is an IgG1, an IG2 or IgG4 antibody of human species.

In a further aspect, the invention relates to conjugates of anti-GPR87 antibody (or a fragment thereof) and a therapeutic moiety or a radioactive metal ion. Examples of therapeutic moieties (e.g. cytotoxic or anti-mitotic agents or anti-metabolites or antibiotics), are taxanes, emetine, mitomycin, etoposide, tenoposide, vincristine, vinblastine, colchicin, doxorubicin, daunorubicin, dihydroxy anthracin dione, mitoxantrone, mithramycin, methotrexate, 6-mercaptopurine, 6-thioguanine, cytarabine, 5-fluorouracil, decarbazine, mechlorethamine, melphalan, carmustine (BSNU), lomustine (CCNU), cyclothosphamide, busulfan, dibromomannitol, streptozotocin, mitomycin C, cis-dichlorodiamine platinum (II) (DDP) cisplatin, daunorubicin and doxorubicin, bleomycin, mithramycin, anthramycin (AMC), maytansines, auristatins (Doronina et al., 2003), calicheamicin and analogs or homologs or derivatives thereof.

Techniques for conjugating a therapeutic moiety to an anti-GPR87 antibody are well known, see, e.g. Arnon et al., 1985; Hellstrom et al., 1987; Thorpe, 1985; Pinchera et al., 1985; Baldwin et al., 1985; and Thorpe et al., 1982. Alternatively, the anti-GPR87 antibody molecule may be conjugated to a biologically active molecule, like cytokines, e.g. IL-2, as described by King et al., 2004.

Alternatively, the anti-GPR87 antibody molecule may be conjugated to RNAse, as described by Newton and Rybak, 2001.

Alternatively, an anti-GPR87 antibody molecule can be conjugated to a second antibody molecule, e.g. an anti-CD3 antibody (in order to target the antibodies to T cells) to form an antibody heteroconjugate as described by Segal in US 4,676,980.

Alternatively to conjugating the anti-GPR87 antibody to a second antibody, a bispecific antibody molecule may be used, which consists of a single antibody molecule with two distinct antigen binding sites recognizing, in addition to the GPR87 epitope, another antigen (Kontermann, 2005). If the second binding site recognizes CD3 or Fc-gamma receptors such antibodies increase involvement of immune cells and thereby encance effector cell mediated anti-tumor activity.

The bispecific antibody molecule may be a diabody (as mentioned above in the context with a divalent antibody), which comprises a heavy (VH) chain variable domain connected to a light chain variable domain (VL) on the same polypeptide chain (VH-VL) connected by a peptide linker that is too short to allow pairing between the two domains on the same chain. This forces paring with the complementary domains of another chain and promotes the assembly of a dimeric molecule with two functional antigen binding sites. Diabodies are further described in EP 404,097 and WO 93/11161.

Anti-GPR87 antibody molecules and their conjugates may be useful for the therapy of human diseases as defined herein, in particular for the therapy of cancers.

Anti-proliferative activity of an anti-GPR87 antibody can be assessed by standard methods, e.g. by determining the DNA content of treated cells by FACS analysis after DNA stain with propidium iodide, or by determining the metabolic activity of the cells. The pro-apoptotic activity cells can be assessed via determination of apoptosis markers (e.g. CaspGLOW^{™} Fluorescein Active Caspase staining kit) and subsequent FACS analysis. The ability of a particular anti-GPR87 antibody to mediate lysis of tumor cells by complement activation and/or ADCC can also be assayed by standard methods: for example, the tumor cells are grown and labeled *in vitro;* the antibody is added to the tumor cell culture in combination with either serum complement or immune cells which may be activated by the antigen antibody complexes. Cytolysis of the target tumor cells is detected by the release of label from the lysed cells.

An antibody that shows anti-tumor activity, e.g. via an anti-proliferative or pro-apopototic activity or by activating complement or mediating ADCC *in vitro,* can then be used therapeutically.

The pharmaceutical composition of the invention may contain, as the active ingredient, an anti-GPR87 antibody molecule of any type that binds to GPR87 and exerts the desired anti-tumor effect, as described above. It is useful for the treatment of disorders in which GPR87 is involved, in particular for the treatment of cancer.

Cancers are generally classified in two ways: by the type of tissue in which the cancer originates (histological type) and by primary site, or the location in the body where the cancer first developed. The most common sites in which cancer develops include the skin, lungs, female breasts, prostate, colon and rectum, cervix and uterus.

The anti-GPR87 antibodies of the invention, as well as other inhibitors of GPR87 function like small molecule inhbitors, are useful in the treatment of a variety of cancers, including but not limited to the following:
- brain related cancer such as adult brain tumor, childhood brain stem glioma, childhood cerebellar astrocytoma, childhood cerebral astrocytoma/malignant glioma, childhood ependymoma, childhood medulloblastoma, childhood supratentorial primitive neuroectodermal tumors, childhood visual pathway and hypothalamic glioma and other childhood brain tumors;
- breast cancer;
- digestive/gastrointestinal related cancer such as anal cancer, colon cancer, esophageal cancer, gallbladder cancer, pancreatic cancer, rectal cancer, and small intestine cancer;
- genitourinary related cancer such as trans cellular bladder cancer, penile cancer, prostate cancer, transitional cell renal pelvis and ureter cancer, testicular cancer, and urethral cancer;
- germ cell related cancer such as childhood extracranial germ cell tumor, extragonadal germ cell tumor, ovarian germ cell tumor and testicular cancer;
- gynecologic related cancer such as cervical cancer, endometrial cancer, gestational trophoblastic tumor, ovarian epithelial cancer, ovarian germ cell tumor, ovarian low malignant potential tumor, uterine sarcoma, vaginal cancer and vulvar cancer;
- head and neck related cancer such as hypopharyngeal cancer, laryngeal cancer, lip and oral cavity cancer, metastatic squamous neck cancer with occult primary, nasopharyngeal cancer, oropharyngeal cancer, paranasal sinus and nasal cavity cancer, parathyroid cancer and salivary gland cancer;
- hematologic/blood related cancer such as lymphomas, such as AIDS-related lymphoma, cutaneous T-cell lymphoma, adult Hodgkin's lymphoma, childhood Hodgkin's lymphoma, Hodgkin's lymphoma during pregnancy, mycosis fungoides, adult non-Hodgkin's lymphoma, childhood non-Hodgkin's lymphoma, non-Hodgkin's lymphoma during pregnancy, primary central nervous system lymphoma, Sezary syndrome, cutaneous T-cell lymphoma and Waldenström's macroglobulinemia and other hematologic/blood related cancer such as chronic myeloproliferative disorders, multiple myeloma/plasma cell neoplasm, myelodysplastic syndromes and myelodysplastic/myeloproliferative diseases;
- lung related cancer such as non-small cell lung cancer;
- musculoskeletal related cancer such as Ewing's family of tumors, osteosarcoma, malignant fibrous histiocytoma of bone, childhood rhabdomyosarcoma, adult soft tissue sarcoma, childhood soft tissue sarcoma and uterine sarcoma;
- neurologic related cancer such as adult brain tumor, childhood brain tumor, brain stem glioma, cerebellar astrocytoma, cerebral astrocytoma/malignant glioma, ependmoma, medulloblastoma, supratentorial primitive neuroectodermal tumors, visual pathway and hypothalamic glioma and other brain tumors such as neuroblastoma, pituitary tumor and primary central nervous system lymphoma;
- respiratory/thoracic related cancer such as non-small cell lung cancer, malignant mesothelioma, thymoma and thymic carcinoma;
- skin related cancer such as cutaneous T-cell lymphoma, Kaposi's sarcoma, Merkel cell carcinoma and skin cancer.

GPR87 inibitors, e.g. anti-GPR87 antibodies and small molecule inhibitors, are particularly useful for treatment squamous cell carcinomas and metastases derived from such carcinomas. Examples for squamous cell carcinomas and metastases, respectively, are HNSCC (head and neck squamous cell carcinoma), cervical cancer, esophagal cancer, larynx carcinoma, lung cancer, mycosis fungoides and lymph node metastases.

Apart from being applied in therapy, anti-GPR87 antibody molecules can be used diagnostically, e.g. to monitor GPR87 levels in blood or tissues as part of a clinical testing procedure, e.g. to, determine or predict the efficacy of a given treatment regimen. Detection can be facilitated by coupling the anti-GPR87 antibody molecule to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin. Examples of suitable radioactive labels include ¹²⁵I, ¹³¹I, ³⁵S, ³H or ⁹⁰Y.

Furthermore, an anti-GPR87 antibody molecule can be used to detect the GPR87 protein (e.g. in a cellular lysate or cell supernatant) in order to evaluate the abundance and pattern of expression of the GPR87 protein, e.g. to determine the protein localization in different human/mouse tumors and normal tissues to confirm the relevance of endogenous GPR87 for tumorigenesis.

Furthermore, an anti-GPR87 antibody molecule (e.g. monoclonal antibody) is useful for isolating the GPR87 protein, e.g. by affinity chromatography or immunoprecipitation. An anti-GPR87 antibody can facilitate the purification of natural and recombinantly produced GPR87.

Also, an anti-GPR87 antibody molecule may be useful for monitoring a patient's response to a cancer drug, in the case that GPR87 serves as a surrogate marker if an administered compound and a chosen dose and treatment schedule have reached levels sufficient to inhibit the expression of GPR87.

Depending on the indication, anti-GPR87 antibody molecules may be combined with other agents, e.g. anti-cancer agents such as conventional chemotherapeutics like the drugs described above in the context with antibody conjugates, in particular taxanes, alkylating compounds, platinum compounds, topoisomerase inhibitors or antimetabolites, calicheamicin, with hormones or anti-hormones with antibodies like Herceptin and/or with immunostimulatory molecules, in particular cytokines like Interleukin-2, granulocyte colony-stimulating factor (G-CSF), IL-12 and/or radiotherapy.

The present invention also relates to vectors which comprise one or more polynucleotides encoding human GPR87 and/or a GPR87-G-alpha fusion protein, host cells which are genetically engineered with such vectors, and the production of GPR87 and/or GPR87-G-alpha fusion polypeptides by recombinant techniques. Cell-free translation systems, such as the commercially available *in vitro* translation kits from Roche (wheat germ translation) or Ambion Inc. (Retic Lysate IVT Kit) can also be employed to produce GPR87 and/or GPR87-G-alpha fusion polypeptides using RNAs derived from the DNA constructs of the present invention.

For recombinant production according to methods well known in the art, host cells are genetically engineered to incorporate expression systems for GPR87 and/or GPR87-G-alpha fusion polypeptides. Introduction of polynucleotides into host cells can be effected by methods described in many standard laboratory manuals (e.g. Sambrook, 1989) such as calcium phosphate transfection, DEAE-dextran-mediated transfection, microinjection, cationic lipid-mediated transfection, electroporation (e.g. Amaxa), transduction, ballistic introduction or infection.

As described above, GPR87-G-alpha fusion polypeptides are preferably, but not limited to, fusion polypeptides of GPR87 with GNAQ, GNASL, GNASS and GNA15.

Representative examples of suitable hosts include different strains of *Escherichia coli,* such as JM109, JM110, XL1 blue; fungal cells, such as yeast cells and *Aspergillus* cells; insect cells such as *Drosophila* S2 and *Spodoptera* Sf9 cells; mammalian cells such as CHO, COS, HeLa, 3T3, HEK 293 and HN5 cells including normal primary cell lines and low-passage number cell lines derived from cancer patients.

A great variety of expression systems can be used. Such systems include, among others, chromosomal, episomal and virus-derived systems, vectors derived from bacterial plasmids, from bacteriophages, from transposons, from yeast episomes, from insertion elements, from yeast chromosomal elements, from viruses such as baculoviruses, papova viruses, such as SV40, vaccinia viruses, adenoviruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression, such as Tet-inducible expression cassettes (Baron and Bujard, 2000). Generally, any system or vector suitable to maintain, propagate or express a polynucleotide to produce a polypeptide in a host may be used. The polynucleotide encoding GPR87 and/or GPR87-G-alpha fusion polypeptide may be inserted into an expression system by any of a variety of well-known and routine techniques, such as, for example, those set forth in Sambrook, 1989. For secretion of the translated protein into the lumen of the endoplasmic reticulum into the periplasmic space or into the extracellular environment, appropriate secretion signals may be incorporated into the polynucleotide encoding GPR87 and/or the GPR87-G-alpha fusion polypeptide. These signals may be endogenous to the polypeptide or they may be heterologous signals.

If the GPR87 polypeptide and/or the GPR87-G-alpha fusion polypeptide is to be expressed in a cell for use in an GPR87 assay, it is generally preferred that the polypeptide is produced such that it is presented at the surface of the cell. This can be achieved by expressing a fusion protein consisting of a GPR87 polypeptide and/or GPR87-G-alpha fusion polypeptide that is N-terminally fused to a leader peptidase signal. Suitable expression vectors are commercially available, e.g. pSecTag2(abc) from Invitrogen that utilizes the Igκ-leader sequence for secretion.

If, instead of using whole cells, membrane fractions are used to enrich the cellular compartment carrying GPR87 (hGPR87), the cells are lysed after harvest, because GPR87 is not secreted to the surrounding medium. For high-level fermentation, solubilization and purification, expression may be performed in *Pichia pastoris* (Schiller et al., 2001; Zeder-Lutz et al., 2006).

GPR87 polypeptides can be recovered and purified from recombinant cell cultures by well-known methods as e.g. described by Schindler and Doods, 2002, including ammonium sulfate or ethanol precipitation, acid extraction, anion or cation exchange chromatography, phosphocellulose chromatography, hydrophobic interaction chromatography, affinity chromatography. Most preferably, high performance liquid chromatography is employed for purification. Well known techniques for refolding proteins may be employed to regenerate active conformation when the polypeptide is denatured during isolation and/or purification.

The above-defined expression systems ae suitable for expression of both human GPR87 and homologues thereof, in particular mammalian homologues like murine GPR87.

In a further aspect, the present invention relates to methods for determining a compound's effect on GPR87 activation ("GPR87 assay methods" as defined above).

When employing GPR87 in such assay method, it has to be considered that the functional responses of GPCR activation on ligand binding strongly depend on the identity of the G-alpha subunit contained in the receptor-coupled heterotrimeric G-protein complex, respectively.

Thus, in the GPR87 assay methods of the invention, fusion polypeptides in which the C-terminus of human GPR87 is fused to the N-terminus of a specific G-alpha subunit are used to couple human GPR87 activation to a certain functional response, e.g. alteration of intracellular cAMP or Ca²⁺ levels, phosphorylation of MEK or MAPK, internalization of GPR87 via ß-arrestin, or signals generated by reporter systems.

This strategy allows linking human GPR87 activation (or inhibition, respectively) that occurs upon binding of a test compound to GPR87 to a certain signaling pathway.

It could be shown that the level of functional response of adenylate cyclase (intracellular cAMP levels), which is specifically activated by GTP-bound (activated) G-alpha proteins, is dramatically enhanced, by using a GPCR-G-alpha fusion protein. By fusing the β2-adrenoceptor C-terminus in frame with the N-terminus of G-alpha proteins, the promiscuity of G-alpha proteins towards different GPCRs could be avoided (Seifert et al., 1999a). As described above, the GPR87-G-alpha fusion polypeptides of this invention include, but are not limited to, fusion polypeptides of human GPR87 with GNAQ, GNASL, GNASS and GNA15.

In a further aspect, the present invention relates GPR87 assay methods of the invention as defined herein that employ human GPR87 and/or a GPR87-G-alpha fusion polypeptide are useful for determining whether a test compound
a) binds to and activates hGPR87 and/or a GPR87-G-alpha fusion polypeptide (agonist), and/or
b) inhibits the generation of intracellular signals generated upon binding of endogenous ligands and thus activation of human GPR87 by
   i. binding to the receptor binding site of the endogenous ligand, or by
   ii. allosteric hinderance, or
c) inhibits the interaction of GPR87 with its endogenous receptor ligands (antagonists) or
d) acts intracellularly on a signal in the cascade transduced upon activation of GPR87 upon ligand binding;
wherein cells expressing on their surface (either endogenously or upon transfection with an appropriate DNA molecule) human GPR87 and/or a GPR87-G-alpha fusion polypeptide, or a membrane fraction of such cells, are incubated with said test compound and said test compound's ability to modulate GPR87 activity as defined in a) to d) is determined.

Thus, GPR87 assay methods employing GPR87 and/or GPR87-G-alpha fusion polypeptides may be used to assess in for example cells or cell-free membrane preparations, binding of small molecules and other binders contained in e.g. chemical libraries and natural product mixtures. Binders may be also be natural ligands or structural or functional mimetics thereof (see Coligan et al., 1991).

The use of GPR87-G-alpha fusion polypeptides in the assay methods of the invention allows coupling of the GPR87 signal transduction upon receptor activation to a G-alpha specific signaling pathway and thus to a functional cellular response. The different GPR87 assay methods described herein are therefore based on the utilization of a GPR87-G-alpha fusion polypeptide.

The four different GPR87-G-alpha fusion polypeptides (GPR87-GNAQ, GPR87-GNASS, GPR87-GNASL, and GPR87-GNA15) of this invention allow the coupling of GPR87 activation signals towards specific signaling pathways. The specificity of the generated signals (e.g. GPR87-GNAQ coupling to an alteration of the intracellular Ca²⁺ levels, GPR87-GNASL coupling to an increase in cAMP concentration) can be harnessed for screening purposes. The use of different GPR87-G-alpha fusion polypeptides will additionally enable to distinguish between agonists/antagonists acting directly on GPR87 polypeptide and/or the downstream signal pathways of the screening method. The effect of agonists and/or antagonists acting directly on GPR87 polypeptide will result in identical responses, independent of the applied G-alpha-subunit and the coupled downstream signaling pathway. In contrast, compounds positively or negatively interfering with the downstream signaling cascade will lead to different signals depending on the G-alpha-subunit used. The fusion proteins of the invention also allow to identify the native ligands of GPR87. Once the native ligand is known, the native (endogenous) G-alpha-subunit can also be identified by comparing the signals generated in cells expressing the GPR87-fusion polypeptides with those expressing wilde type GPR87.

In general, cellular GPR87 assay procedures are based on appropriate cells that express GPR87 on the cell surface. Such cells include cells from mammals, yeast, *Drosophila* or *Escherichia coli.* In particular, a polynucleotide encoding GPR87 and/or a GPR87-G-alpha fusion polypeptide is employed to transfect cells. Alternatively, cells may be used that express GPR87 endogenously, e.g. tumor cell lines like HN5.

In first instance, it may be useful to provide the expressed GPR87 polypeptide together with its endogenous ligand to reconstitute the expected functional response in the assay format of interest. The expressed GPR87 is then brought into contact with a test compound to detect its binding and ability to stimulate or inhibit receptor activation, respectively, by monitoring a functional response, as described below. In some embodiments, the GPR87 assay procedure is based on the use of cells which express a GPR87 polypeptide (for example, hGPR87-transfected CHO cells) and in which, upon GPR87 activiation or inhibition, a second messenger response (signal) can be measured, such as a change in the intra- and extra-cellular pH value or alterations in the level of a second messenger molecule in the signal transduction pathway that is caused by an increase or inhibition of receptor activation. A detailed description and summary of useful methods is given by Schnitzer and Sommergruber (2006) and references cited therein. In general, these methods involve the use of cells which transiently or stably express the GPR87 polypeptide, preferably hGPR87 and/or the respective GPR87-G-alphafusion polypeptide, for example, *Xenopus* oocytes or cell lines such as CHO, HEK 293 (embryonic kidney), HMEC (epithelial), HIVE-55 (endothelial), HIVS-125 (smooth muscle), and tumor cells (e.g. HN5) but are not limited to these cell types and cell lines. The transfected *Xenopus* oocytes or cell lines are then incubated with the native (endogenous) receptor ligand, such as dATP, dAMP, or UTP and the test compound. Inhibition or activation of the receptor is then determined by measuring alterations of a signal that reflects changes in the receptor-coupled signal transduction pathway, such as changes in the cAMP or intracellular calcium levels or changes in the intra- and extracellular pH value. For example, methods to measure changes in intracellular calcium concentration in a high throughput mode in microtiterplates have been already developed (Stables et al., 1997; Coward et al., 1999).

In other embodiments, the assay method of the invention is based on a binding assay which involves, in the presence or absence of a test compound, determination of inhibition of binding of labeled GPR87 ligands, such as dATP, dAMP, or UTP to cells which present GPR87 on the surface, or cell membranes containing GPR87 such as membrane fractions of CHO-K1 cells stably expressing GPR87 are commercially available (Upstate Biotechnology).

Such a method involves transfecting a eukaryotic cell with DNA encoding the GPR87 polypeptide and/or GPR87-G-alpha fusion polypeptides in a way that the cell expresses the receptor on its surface. The cell, or the membrane fraction, respectively, is then incubated with the test compound, e.g. a potential antagonist, in the presence of a labeled ligand, such as dATP, dAMP, or UTP. The ligand is labeled, e.g. with radioactivity or fluorescence. The amount of labeled ligand bound to the receptor is measured, e.g., by measuring radioactivity associated with transfected cells or membrane fractions. If the test compound binds to the receptor, binding of labeled ligand to the receptor is inhibited, which results in a reduction of labeled ligand bound to the receptor. The same technique can be used to screen for agonists. Whether a compound acts as an agonist or an antagonist can be determined by analyzing the downstream signaling events such as the increase of cAMP. In the case of activation cAMP levels are increasing in the presence of an inhibitor a decrease is observed.

Since the activity of GPCRs, including GPR87, initiates signaling events in the cell that may act on response elements contained in a reporter gene construct and are thus are detectable via expression of a reporter molecule, the present invention relates, in a further aspect, to a reporter-based GPR87 assay.The GPR87 assay of the invention is a reporter-based assay that measures the activity of a reporter gene product in cells genetically engineered to express the reporter molecule under the control of a regulatory sequence containing a specific promoter and associated response elements that respond to specific signals, e.g. types of stress or molecules of a specific signal transduction cascade.

The GPR87 reporter assay of the invention works according to the same principle as the method described in WO 93/11257, which is incorporated by reference herewith. The assay method of WO 93/11257 determines whether a compound has a modulating effect on a receptor-dependent signal transduction pathway in test cells that are transformed with a DNA coding for a receptor coupled to the phospholipase effector system, in particular a G protein-coupled receptor, and a reporter gene with a regulatory sequence functionally linked thereto that is sensitive to the IP₃/DAG concentration (IP₃ and DAG being second messenger molecules generated upon activation of the receptor-coupled signaling cascade).

Specificity and sensitivity of the system are controlled by the choice of the promoter and the transcriptional control region, which determine the basal expression level and the degree of transcriptional response. Other factors influencing the sensitivity of the system are the stability of the reporter molecule in the cell and the dynamic range of the reaction. A short half-life of the reporter molecule is important for minimal basic accumulation of the signal and hence a high dynamic range. Enzymes as reporters have the advantage of signal amplification which improves sensitivity and dynamic range. The ideal reporter enzyme is not endogenously produced in the cell, thus eliminating background expression. Examples for frequently used reporter genes that are suitable in the method of the present invention are secreted alkaline phosphatase, ß-galactosidase, GFP, luciferase and β-lactamase out of a larger number of available genes, which have been reviewed in Suto and Ignar (Suto and Ignar, 1997). A particularly useful reporter is SEAP (secreted placental alkaline phosphatase; Berger et al., 1988), which is a mutated form of the human placental enzyme, can easily be monitored by colorimetric, fluorescent and chemiluminescent detection systems, e.g. a chemiluminescence assay based on the use of 1,2-dioxetane substrates.

Another useful reporter is ß-galactosidase, commonly referred to as lacZ, for which different substrates have been developed (Jain and Magrath, 1991; Naylor, 1999) including those for fluorometric analysis (Labrousse et al., 1982) or laser-induced fluorescence detection of ß-gal protein levels. A luminescence-based assay even lowers the detection limit of ß-gal to ~2fg and is about three times more sensitive than the above mentioned fluorescence assays (Bronstein et al., 1996). Another possible reporter gene is green fluorescent protein (GFP). GFP from e.g. the jellyfish, *Aequoria victoria,* transforms the blue chemiluminescence of aequorin into green fluorescent light with an emission maximum at 508 nm (Cubitt et al., 1995). GFP has been extensively studied by mutational analysis leading to different variants of this protein in respect to increase in thermostability, intensity, and shifts within the fluorescence spectra (Tsien, 1998). The universal application of GFP to highly different organisms and the fact that it requires no substrate for its activity makes it the reporter of choice in eukaryotes.

Due to several advantages, the luciferase gene from *Photinus pyralis* (de Wet et al., 1987) is still the most commonly used reporter gene. In a two-step reaction the enzyme catalyses the oxidation of luciferin which results in an emission of a short flash of light at 550-570 nm. Optimized reaction buffers have lead to a prolonged half-life of the signal to a so-called "glow reaction" which is stable for more than 1 hour. Appropriate buffers are commercially available (e.g. Dual-Glo Luciferase Assay System from Promega). The assay protocol is very simple and only requires the addition of the reaction buffer which contains ATP and luciferin and lyses the cells. Luciferase is not endogenously expressed and has a short half-life of 3-4 hours (Himmler et al., 1993). Hence there is no intrinsic background in cells and the short half-life allows to track reduction of expression efficiently. Another example for a reporter is *Renilla* luciferase from sea pansy *Renilla reniformis* The activity of *Renilla* luciferase can be monitored in live cells, thus eliminates the need for lysis of cells and allows for multiple measurements. Recently, a novel *Renilla* luciferase substrate, EnduRen^{™} has been become available from Promega, which is significantly more stable and should allow prolonged real-time measurements. *Renilla* luciferase is very useful in a dual reporter assay together with beetle luciferase, where the activity of both enzymes are measured in two consecutive steps. Dual bicistronic reporter gene assays with simultaneous measurement of the activities of both reporter genes can also be generated by using click beetle luciferase as the second reporter gene. Several luciferases from the click beetle *Pyrophorus plagiophtalamus* (Wood et al., 1989; Stolz et al., 2003) are available for this purpose. Dual bicistronic reporter assays allow to monitor the activity of two distinct reporter genes driven by different promoter elements in one experiment. This system can be used to measure the activity of two different signaling pathways (elicited by activation of one receptor) or of two receptors at the same time. This type of assay system can also be applied for a ratiometric analysis of the activity of two differentially regulated reporter genes and thus normalize the activity of one reporter gene to the second one (for example, one reporter gene is driven via the GPR87-specific pathway the other via a control GPRC such as P2Y14). This type of assay allows, by normalization to the number of cells per well, control of experimental variability (resulting from e.g. edge effects) or of viability (which may be affected by non-specific toxicity of tested compounds.

Another reporter enzyme useful in the GPR87 assay method of the invention is bacterial ß-lactamase (Moore et al., 1997). The ß-lactamase cell-based assay system couples molecular and biological events to a fluorescence resonance energy transfer (FRET)-based detection method in single live cells (GeneBLAzer^{™} technology, Invitrogen). Several favourable properties (e.g. no background, high sensitivity) and miniaturization beyond the 384-well plate format to 3456-well format (Kornienko et al., 2004) make the β-lactamase a very attractive reporter gene, especially for screening purposes.

In cases where the activation of one specific signaling cascade is under investigation (as it is the case with GPR87), the use of a synthetic regulatory region containing the respective response element is the preferred approach, as described in WO 93/11161. To enhance the effect of the second messenger on the regulatory sequence, it preferably contains several copies of the response element. A whole variety of promoter elements that are responsive to the activation of different second messenger pathways are available. One such response element is selected from CREs (cAMP responsive elements, e.g. described in WO 93/11161, Montminy et al., 1990; George et al., 1997). GPCRs coupling to Gαᵢ and Gas can be investigated by CREs; which bind CREB (CRE binding protein) phosphorylated by PKA (Himmler et al., 1993). Those coupling to Gα_{q} signal through calcium which in turn activates the NFAT response element (Boss et al., 1996).

The promoter region of the ICAM-1 receptor, which confers response to several signaling pathways (Voraberger et al., 1991; Stratowa and Audette, 1995), can also be used. It was successfully used for the construction of reporter gene assays for different GPCRs, e.g. NK2 and 5-HT₂ (Weyer et al., 1993; WO 93/11161). Such reporter gene technology was also applied to identify modulators of orphan GPCRs (Cacace et al., 2003). An artificial multiple regulatory element containing the major response elements, through which different G-proteins can signal, allows to set up a reporter assay without knowledge of the coupling mechanism of the orphan GPCR (Chin et al., 2003) such as GPR87. It is therefore necessary to test compounds in a control cell line in parallel. Ideally this is the same cell line as the test cell line with the difference that the reporter gene is induced differently. Another option is to use a different cell line of the same origin as the target cell line. In the case of GPCRs, it should express the same reporter that is sensitive to the same signaling cascade, with the difference that the signaling casdace is coupled to activation of another receptor GPCR or with the difference that the reporter gene expression is driven by a promoter that is regulated by a signaling cascade that is different from the signaling cascade of interest.

In another embodiment, the GPR87 assay method of the invention is based on the determination of the intracellular Ca²⁺ release. As for the methods described above, it involves the use of mammalian cells (CHO, HEK 293, Xenopus oocytes, RBL-2H3, etc.) which are transfected to express GPR87 and/or the GPR87-G-alpha fusion polypeptide. The cells are loaded with an indicator dye that produces a fluorescent signal when bound to calcium, e.g. FLUO-3 and FLUO-4 (Gee et al., 2000), or a recently developed "no-wash calcium assay kit" (Molecular Devices). In the presence of a receptor ligand, such as dATP, dAMP or UTP, and in the presence or absence of the test compound, the fluorescent signal is measured over a defined period of time using, for example, a fluorescence spectrophotometer or a fluorescence imaging plate reader. A decrease or increase in the fluorescence signal indicates that a compound is a potential antagonist or agonist, respectively, for GPR87. The assay may be performed in a cell-based microarrayed screening format (Gopalakrishnan et al., 2003).

In another embodiment, the GPR87 assay method of the invention is based on a similar principle, i.e. the measurement of the change in intracellular calcium concentration. As in the other assay methods, GPR87 and/or a GPR87-G-alpha fusion polypeptide is overexpressed in the test cell line. In one of the methods useful to measure changes in intracellular calcium concentration, the test cells are contransfected with apoaequorin. Coelenterazine is added as cofactor to the reaction. Binding of calcium leads to an oxidation reaction generating coelenteramide, CO₂ and luminescence at 469 nm (Brini et al., 1995). Another technology uses the addition of fluorescent calcium indicators, which change fluorescence intensity dependent upon calcium concentration such as FLUO-3 and FLUO-4 (Gee et al., 2000), or a novel "no-wash calcium assay kit" (Molecular Devices). The convenience of the second approach is that is does not require the cotransfection of apoaequorin. This together with the development of a powerful fluorescent imaging plate reader (FLlPR, Molecular Devices), equipped with an argon laser, a cooled CCD camera, fast simultanoues liquid transfer, plate handling capabilities and data evaluation software, allows to run homogeneous kinetic cellular fluorescent assays with time resolution of seconds down to 1536-well plate formats in high throughput. The fluorescence or bioluminescence-based methods listed above have been described to be suitable for a high throughput mode in microtiterplates, e.g. Stables et al., 1997; Coward et al., 1999; Le Poul et al., 2002. An advantage of this approach is that the changes in concentration can be monitored very fast within seconds or minutes after activation of the signaling cascade and the measured interaction point is further upstream than a reporter gene response. Therefore, general toxic effects of compounds and unspecific interactions at the different steps of the signaling cascade are reduced. The activity of a GPR87-G-alpha fusion polypeptide such as GPR87-Gq signals through the phospholipase C cascade, which subsequently leads to changes in the intracellular calcium concentration and can therefore be monitored with by these approaches (Jerman et al., 2001). It has thus made this technology the first choice for high throughput applications in the field of intracellular calcium measurement. Such an approach will be very useful to identify and characterize native ligands of GPR87 and to screen for novel synthetic inhibitory and activating compounds for pharmaceutical development.

In another embodiment, the GPR87 assay of the invention is based on the detection of a compound's effect on conformational changes (e.g. oligomerisation, interaction with ß-arrestin and internalization etc.) of GPR87 upon activation by means of FRET (fluorescence resonance energy transfer) or BRET (bioluminescence resonance energy transfer). The general principle of these technologies is based on the transfection of the cells with genes of the interacting proteins fused to two different fusion partners between which resonance energy transfer or enzyme complementation occurs if both are in close proximity.

FRET may be applied to investigate conformational changes within GPR87 upon agonist binding by incorporating two different color variants of GFP, CFP and YFP into different parts of the receptor. Vilardaga and colleagues have already used this method to analyze activation of GPCRs (Vilardaga et al., 2003).

In contrast to FRET, where the donor molecule is fluorescent and must be excited with monochromatic light, BRET uses a bioluminescent donor molecule, which does not require an external light source. Most frequently used donor-acceptor pairs are *Renilla* luciferase and different color variants of green fluorescent protein (GFP) from *Aequoria victoria* which have been developed during the last few years.

BRET has been used successfully *in vitro* and *in vivo* to identify and investigate molecular interactions, e.g. hetero- and homodimerization of GPCRs (Michelini et al., 2004) or the interaction of GPCRs with ß-arrestin (Angers et al., 2000), which only binds to the activated form of the receptor. A recent overview over the application of BRET and FRET in the area of GPCR complex formation is given by Pfleger and Eidne (Pfleger and Eidne, 2003). Following the protocol of Angers et al., 2000, a similar approach might be performed for GPR87.

Another GPR87 assay of the invention is based on agonist-mediated ß-arrestin translocation by GPCR87. Based on this approach a universally applicable screening assay has been developed (Vrecl et al., 2004), which is based on enhancing the signal generated from the interaction between a G-protein-coupled receptor (GPCR) and beta-arrestin 2 (beta-arr2). Apart from being independent of the signaling pathway, this approach has several additional advantages over the traditional reporter-based assays. First of all it is a ratiometric measurement and thus not dependent on the number of cells per well, which significantly reduces variability. Due to this and since it directly monitors the interaction of the targets of interest, the number of false positive hits is reduced as compared to reporter gene assays, which measure the signal of a signaling cascade with many more steps. To establish an appropriate high quality GPR87 assay, which is suitable for the HTS format,, different combinations of N- and C-terminal fusion constructs of beta-arrestin 2 are tested in preliminary assays since the resonance energy transfer efficiency is dependent on the proper orientation of donor and acceptor molecule, an unfavorable geometry can lead to a poor signal (Vrecl et al., 2004).

In a further aspect, the present invention relates to a GPR87 assay that is useful for determining whether a compound has a modulating effect on a GPR87-coupled signal transduction pathway, i.e. a compound that does not act by directly modulating GPR87 activation, but that acts on a signaling molecule further downstream in the GPR87-coupled cascade.

Again, this involves the use of mammalian cells (CHO, HEK293, *Xenopus* Oocytes, RBL-2H3, etc.) which are transfected to express both the GPR87 and/or a GPR87-G-alpha fusion and a reporter gene (e.g. luciferase or beta-galactosidase) whose expression is coupled to the activation of GPR87, e.g. by being under the control of the promoter of a transcription factor at the end of the GPR87 signaling cascade, e.g. a STATpromoter).

In another embodiment, the GPR87 assay of the invention is in the form of a translocation assay, in which, upon GPR87-coupled activation, the translocation of a transcription factor from the cytoplasm to the nucleus is determined, e.g. in a high content screening. The potential of cell-based translocation assays for GPCR discovery has recently been described for CXCR4 (Granas et al., 2005). Furthermore, such an assay format allows many other parameters to be tested in parallel e.g., induction of apoptosis, membrane stability, nuclear fragmentation, determination of the intra- and extracellular pH-value, changes in the redox potential to name but a few (Ghosh et al., 2000; Ghosh et al., 2005; Cellomics Inc.). The reporter signal is measured under similar conditions as described above after a defined period of time. The signal can be measured using a luminometer, spectrophotometer, fluorometer, or any other instrument appropriate for the signal generated by the specific reporter gene product. Reduction of the signal indicates that a compound might be a potential antagonist for the receptor.

In another aspect, the GPR87 assay method of the invention is based on determining an decrease or increase of cAMP level and/or inhibition or activation of adenylate cyclase mediated by GPR87 and/or a GPR87-G-alpha fusion polypeptide. Again, such an approach depends on transiently or stably transfected cells expressing GPR87 and/or GPR87 and/or a GPR87-G-alpha fusion polypeptide on the cell surface. These cells are exposed to test compounds in the presence of endogenous ligands, such as dATP, dAMP, or UTP. For example, the change the cAMP levels is then measured over a defined period of time, for example, by radio-immuno or protein binding assays (e.g. using Flashplates or a scintillation proximity assay). Changes in cAMP levels can also be determined by directly measuring the activity of the enzyme, adenylate cyclase, in cell lysate preparations. If the potential antagonist binds the receptor, and thus inhibits GPR87 polypeptide and/or GPR87-G-alpha-fusion polypeptides-ligand binding, the levels of GPR87 polypeptide- and/or GPR87-G-alpha-fusion polypeptides-mediated cAMP, or adenylate cyclase activity, will be reduced; in the case of an agonist activity will be found to be increased.

The GPR87 assay methods of the present invention are also useful for identifying new endogenous ligands not yet known to be capable of binding to an GPR87 polypeptide and/or GPR87-G-alpha-fusion polypeptides. Any of the assay methods described above may be used to identify new ligands.

As noted above, a potential inhibitor identified or characterized in any of the above-described methods is a small molecule which binds to the GPR87 polypeptide and/or GPR87-G-alpha-fusion polypeptide, making it thereby insensitive to ligands such that normal biological GPR87 activity is blocked. A small molecule identified may also be an allosteric (noncompetitive) inhibitor, which exerts its effect by inhibitors binding to a site other than the active site and render the receptor ineffective. Examples of small molecules include, but are not limited to, small peptides or peptide-like molecules (peptidomimetics) and, in particular, synthetic organic molecules.

In an embodiment of the invention, in parallel to a GPR87 screening, or as secondary screen after such screen, the test compound is subjected to a proliferation and/or apoptosis assay in order to assess its inhibitory effect on tumor cells. Proliferation assays are well known in the art, e.g. based on metabolism, cell number counting, etc. Examples for commercially available proliferation assays are the Alamar Blue assay or the MTS assay. An example for an apoptosis assay is the PARP cleavage assay, which is commercially available; or an assay based on determination of DNA double-strand breaks (e.g. the ApoDirect kit).

In order to explore the *in vivo* activity of GPR-87 inhibitors (in particular, anti-GPR87 antibodies and small molecule inhibitors), well characterized tumor models can be used. Such tumor models can be obtained by routinely available methods: by way of example, human cancer cell lines expressing GPR87 are implanted subcutaneously as xenografts in immunodeficient mice or rats. The candidate inhibitors can be administered either intravenously, intraperitoneally or orally. When GPR87 inhibitors are intended for combination therapy, e.g. with standard chemotherapy, concomitant as well as sequential administration schedules are explored.

The *in vivo* efficacy of GPR87 inhibitors is first addressed in standard human tumor models in nude mice. Xenografts derived from the HN5 squamous cell carcinoma cell line are established models for the preclinical evaluation of oncology compounds. By way of example, a total of 10⁶ tumor cells is injected subcutaneously into the right flank of nude mice in a final volume of 100 ml. Mice bearing established tumors with an average volume of 50-100 mm³ are randomized into treatment and control groups with at least 10 individuals per group. GPR87 inhibitors can be tested in a variety of schedules and at several dose levels. Statistical evaluation is typically performed at the end of the experiment when the average of control tumors reaches a volume of 1250 mm³. The parameters to be included in the evaluation include tumor volumes and body weights. For small organic molecules, it is useful to obtain plasma samples in order to monitor peak levels (Cₘₐₓ) and total exposure (AUC ₀₋₂₄ₕ) of active drug regimens.

A pharmaceutical composition containing, as the active ingredient, a GPR87 inhibitor, e.g. an anti-GPR87 antibody or a small molecule inhibitor, is administered to the patient in therapeutically effective amounts that eliminates or reduces the patient's tumor/metastasis burden. It will normally be administered parenterally, preferably intravenously or subcutaneously. The dose and dosage regimen will depend upon the nature of the cancer (primary or metastatic), the site to which the antibodies are to be directed, the characteristics of the particular immunotoxin (in the case that an antibody conjugate is used), e.g. its therapeutic index, the patient, and the patient's history. The amount of antibody administered will typically be in the range of about 0.1 to about 30 mg/kg of the patient's body weight.

For parenteral administration, the anti-GPR87 antibody molecule (or fragment or conjugate) can be formulated in a unit dosage injectable form (solution, suspension, emulsion) in association with a pharmaceutically acceptable parenteral vehicle. Such vehicles are inherently nontoxic, and non-therapeutic. Examples of such vehicles are water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances that enhance isotonicity and chemical stability, e.g. buffers and preservatives. The antibodies may typically be formulated in such vehicles at concentrations of about 1 mg/ml to 10 mg/ml.

To select an anti-GPR87 antibody molecule for its ability to block the function of GPR87 and to thus block the tumor promoting function of GPR87 and thus are effective in human therapy, xenotransplants of human tumors into nude mice can be used. Such experiments can be done according to standard methods, e.g. by transplanting human tumor cells, which may be derived from a cell line or from a primary tumor, subcutaneously or intravenously into immunodeficient mice, e.g. SCID or nude mice.

The routes for administration (delivery) in patients include, but are not limited to, one or more of: oral (e.g. as a tablet, capsule, or as an ingestable solution), topical, mucosal (e.g. as a nasal spray or powder or aerosol for inhalation), nasal, parenteral (e.g. by an injectable form), gastrointestinal, intraspinal, intraperitoneal, intramuscular, intravenous, intradermal, intratracheal, intracerebroventricular, intracerebral, subcutaneous, ophthalmic (including intravitreal or intracameral), transdermal, rectal, buccal, vaginal, epidural, sublingual. GPR87 inhibitory molecules are preferentially delivered by oral application or nasal spray or powder formulation or aerosol for inhalation.

Potential GPR87 antagonists also include proteins which are closely related (amino acid sequence and/or structure) to the endogenous ligand of the GPR87 polypeptide and/or GPR87-G-alpha-fusion polypeptides, i.e., a fragment of the ligand, which have lost biological function but binding to the GPR87 polypeptide and/or GPR87-G-alpha-fusion polypeptides inhibits activation.

Potential antagonists also include soluble forms of the GPR87 polypeptide and/or GPR87-Galpha-fusion polypeptides, e.g., fragments of the receptor, e.g. from the N-terminus, or peptidomimetics thereof, which bind to the endogenous ligand and prevent the ligand from interacting with membrane bound GPR87 polypeptide and/or GPR87-Galpha-fusion polypeptides.

As described above, the GPR87 assay methods of the invention are not only useful to identify and characterize compounds that directly interact with GPR87 polypeptide and/or GPR87-G-alpha-fusion polypeptides, (agonists and antagonists). By utilizing a reporter gene system that is responsive to downstream signaling events, compounds interfering within the GPR87-driven signaling cascade may be identified and used as potential inhibitors of the GPR87 signal transduction pathway. Targets are, but not limited to, the GPR87-coupled kinase, phospholipase C and D, Ca²⁺-sequestering particles, STATs etc.

Small molecule inhibitors of GPR87 function identified in the GPR87 assays may be used as active ingredients in pharmaceutical compositions for human therapy, in particular cancer, as described above for anti-GPR87 antibodies.

In a further aspect, the invention also encompasses, as GPR87 inhibitors, short interfering RNA (siRNA) molecules that down regulate expression of an GPR87 mRNA by RNA interference ("GPR87 siRNA").
RNA interference refers to the process of sequence-specific post transcriptional gene silencing in animals mediated by short interfering RNAs (siRNA) (Fire et al., 1998). siRNA molecules are short pieces of dsRNA obtained by processing of the dsRNA by Dicer, a ribonuclease III enzyme (Bernstein et al., 2001). Short interfering RNAs derived from Dicer activity are typically about 21-23 nucleotides in length and comprise about 19 base pair duplexes. When dsRNAs are transfected directly into mammalian cells, they activate the interferon system and provoke non-specific gene suppression and cytotoxic response. siRNAs up to 26 nucleotides have proven to be effective at specifically silencing gene expression without causing any interferon response. The siRNA molecules of the present invention encompass human GPR87 siRNAs, which are useful for research to analyze the function of GPR87, and human siRNAs, which may be used in therapy of humans, e.g. for cancer therapy.

Based on the RNA sequence of human GPR87, GPR87 siRNA molecules with the ability to knock down GPR87 activity can be obtained by chemical synthesis or by hairpin siRNA expression vectors (as described by Yu et al., 2002). There are numerous companies that provide the supply of costumer-designed siRNAs on a given RNA sequence, e.g. Ambion, lmgenex, Dharmacon.

The GPR87 siRNAs of this invention may be chemically modified, e.g. as described in US 20030143732, by phosphorothioate internucleotide linkages, 2'-O-methyl ribonucleotides, 2'-deoxy-2'-fluoro ribonucleotides, "universal base" nucleotides, 5-C-methyl nucleotides, and inverted deoxyabasic residue incorporation.

In a further embodiment, the present invention relates to an expression vector comprising a nucleic acid sequence encoding at least one GPR87 siRNA molecule in a manner that allows expression of the nucleic acid molecule, and cells containing such vector.

The GPR87 siRNA molecules of the invention may be delivered by known gene delivery methods, e.g. as described in US 20030143732, including the use of naked siRNA, cationic lipid formulations, liposome formulations including pH sensitive liposomes and immunoliposomes, and bioconjugates including siRNAs conjugated to fusogenic peptides. Delivery of siRNA expressing vectors can be systemic, such as by intravenous or intramuscular administration or by any other means that would allow for introduction into the desired target cell (see US 20030143732).

With regard to short interfering RNA molecules, their use as inhibitors of the function of GPR87, in particular as active ingredients in pharmaceutical compositions for human therapy, in particular cancer, the criteria described above for anti-GPR87 antibodies apply.

### Brief description of the Figures

Figure 1:
   Tumor specific expression pattern of the human GPR87 mRNA transcript
      A: Various tumor cell lines
      B: Box plots and whisker plots of human tumor tissues and their corresponding normal tissues
Figure 2:
   hGPR87: Immunohistochemistry and HE-staining of a human lung squamous cell carcinoma sample (formalin-fixed and paraffin-embedded).
      Antiserum was from Abcam (Rabbit-α-GPR87; directed against the C-terminal intracellular domain).
Figure 3:
   Homology tree of the P2Y subfamily
Figure 4:
   RT-PCR analysis of hGPR87-siRNA knock-down in HN5 cells
Figure 5:
   Analysis of the effects of the siRNA-mediated knock-down of human
   GPR87 protein in head and neck squamous carcinoma cell line HN5
      5A: Western blot analysis
      5B: ApoDirect assay and cell cycle distribution
      5C: Light microscopy
Figure 6:
   Antibody blockade of hGPR87 polypeptide utilizing an antibody specifically recognizing the N-terminal extracellular part of hGPR87. Effect on proliferation and apoptosis of HN5 cells.
      Upper panel: Light microscopy pictures
      Lower panel: Western blot analysis
Figure 7:
   Experiment to rescue the hGPR87 blocking effect in HN5 cells mediated by an anti-GPR87 directed to the N-terminus of hGPR87 polypeptide
      A: Anti-proliferative effect of an anti-GPR87 antibody directed to the N-terminus
      B: Rescue experiment showing concentration dependence of the anti-GPR87 antibody directed to the N-terminus

### Examples

### Example 1

### Expression Profiling Analysis

In total, 687 AffyIDs of 835 manually curated AffylDs, coding for 445 GPCRs are used for this analysis of the human GeneLogic expression database. Cluster analysis (normal and tumor tissues versus transcriptional profile) reveales GPR87 as one GPCR with the most tumor-specific transcription profile (applied filter parameters were: a fold change of at least 4 between normal and tumor tissue, a p-value of less than 0.001 and a tumor-specific expression rate in at least 3 different tumor types).

Based on these analyses a detailed transcription profile is made available utilizing human GeneLogic expression database. The identified human GPR87 transcript is expressed and upregulated predominantly in cancerous tissues, as well as in human cancer cell lines of the corresponding primary tissue.

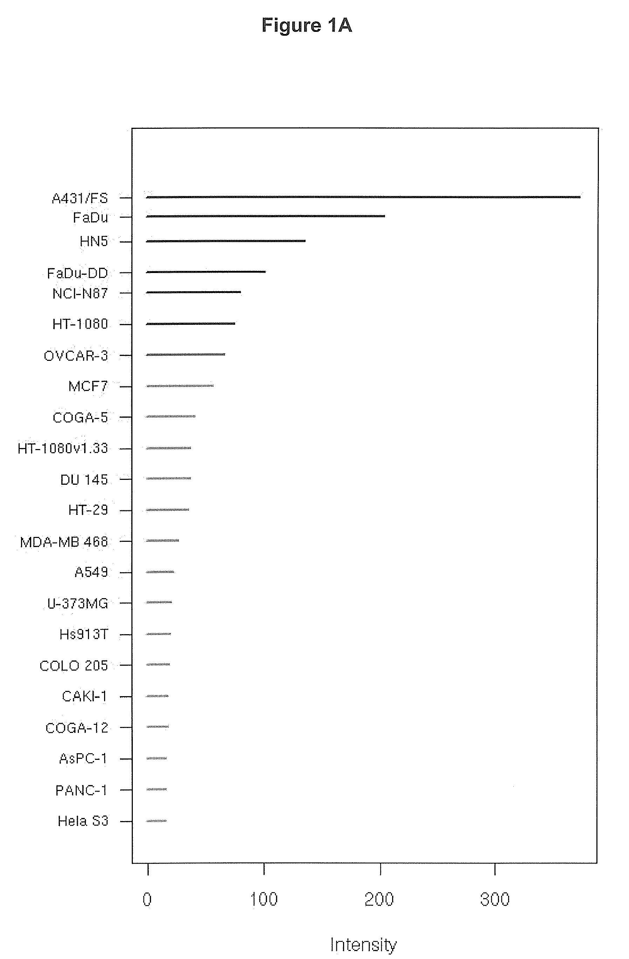

Fig. 1A shows the expression level of GPR87 in various human tumor cell lines. The intensity is presented in arbitrary units. Present calls are indicated by a bold line, absent calls (to be considered as non-transcribed) are indicated as grey. As can be seen from Fig. 1A, HN5 is one of the cell lines that most prominently express GPR87.

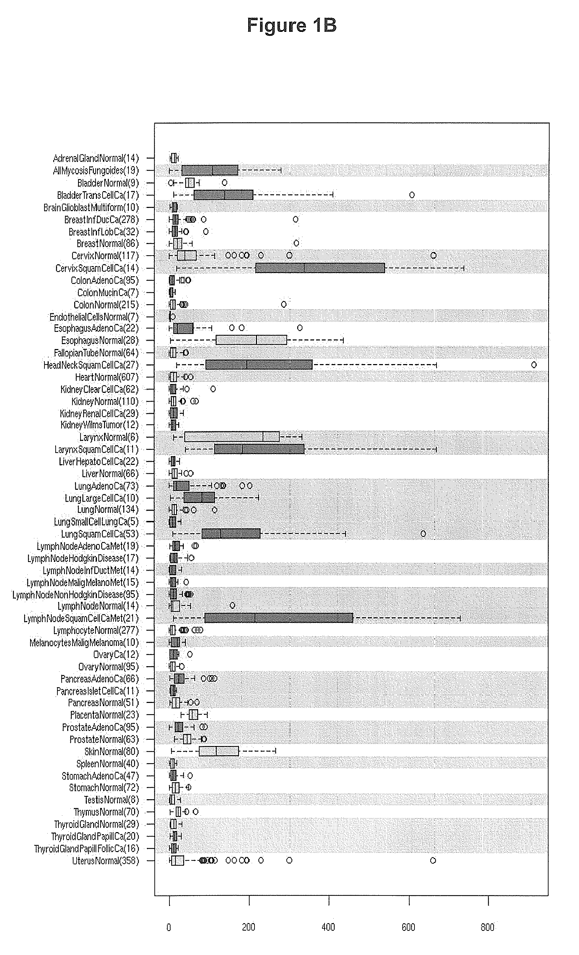

Fig. 1B: Box plots and whisker plots are generated as described (Dolznig et al., 2005; Shen-Ong et al., 2003). The vertical center line indicates the median, the box itself represents the interquartile range (IQR) between the first and third quartiles (red boxes, tumors; green boxes, normal tissue). Whiskers extend to 1.5 times the IQR; the position of extreme values, if above the upper whisker limits, are marked by open circles. Numbers in brackets indicate the sample numbers. The scales show normalized arbitrary intensity values.

In normal tissues expression is limited to esophagus, larynx and skin. hGPR87 mRNA is preferentially expressed in tumors of sqamous cell origin. However, as seen in Fig. 1 B also in samples of lung adeno and breast infiltrating ductal carcinomas, a subpopulation with enhanced levels can be detected. In addition, enhanced transcript levels are also found in the corresponding lymph node metastasis from squamous cell primary tumors (Fig. 1 B).

### Example 2

### Immunohistochemistry of hGPR87 in human lung squamous cell carcinoma

Using a commercially available antibody (Abcam) directed against the C-terminus of hGPR87 protein, a highly tumor-specific staining can be detected in formalin-fixed and paraffin-embedded tissue sections of a lung squamous cell carcinoma sample. Tumor stroma, surrounding normal tissue and infiltrating cells are negative for hGPR87. The specific membrane staining is due to membrane localization of hGPR87, which belongs to the large 7TM protein family of GPCRs.

For immunohistochemistry, an indirect immunoperoxidase assay is used to detect expression of GPR87 in a series of human cancers of diverse histological type. Briefly, 5 µm-thick sections are cut and mounted on poly-(L-lysine)-coated slides. After de-paraffinization and epitope retrieval with Proteinase K in TrisEDTA buffer pH8.0 for 15min at 37°C, the sections are blocked with 10% normal goat serum. Subsequently, the primary antibody against hGPC87 antigen is applied for 1 hour at RT followed by incubation with goat anti-rabbit horseradish peroxidase labeled polymer, Dako Envision System (Dako, Carpinteria, CA) for 30 min at RT. The final reaction product is visualized with DAB (0.06% 3,3'-diaminobenzidine in PBS, 0.003% H₂O₂) for 2-5 min. The slides are then dehydrated in alcohol, and counterstained with hematoxylin. The primary antibodies used are rabbit anti-human GPCR87 ab13945 (Abcam, Cambridge, UK) diluted 1:200 in 2% BSA/PBS and rabbit anti-human GPCR87/GPCR95 SP4578P recognizing the C-terminus (Acris, Hiddenhausen, Germany) diluted 1:200 in 2%BSA/PBS. The specificity of the staining is confirmed by pre-incubating the primary Ab with the immunizing peptide comprising the last 41 amino acids of SEQ ID NO:2. from peptide.de) for 2 hr at RT at a final concentration of 125 µg/ml of Ab which results in no staining of the tumor cells.

### Example 3

### hGPR87 knock-down by siRNA in human head and neck squamous cell carcinoma cell line HN5 induces apoptosis

To study the effect on viability, proliferation, differentiation, apoptosis, and cell cycle regulation, a specific siRNA targeting human GPR87 mRNA is designed and obtained from *Dharmacon RNA technologies.* SEQ ID NO:3 represents the targeted mRNA sequence within the human GPR87 mRNA.

Human HN5 head and neck squamous cell carcinoma cells are grown in DMEM supplemented with 10% fetal bovine serum, 10 mM Hepes, 1 % L-Gluthamine, non-essential-amino-acids, and 1 mM sodium pyruvate (all purchased from Invitrogen/Gibco, Carlsbad, CA, USA). HN5 cells are then transfected with human GPR87 siRNA by DharmaFect as follows: A SMARTpool-plus set of 4 individual siRNAs targeting the hGPR87 mRNA sequence is obtained from Dharmacon (Lafayette, CO, USA). The pool and the individual siRNAs are tested separately for efficient down-regulation of hGPR87 mRNA and protein levels. The siRNA duplex targeting SEQ ID NO:3 shows the most efficient silencing effects. siCONTROL Non-Targeting siRNA #1 is also purchased from Dharmacon (Lafayette, CO, USA). For each siRNA transfection, approximately 2 x 10⁶ cells are plated in a 15 cm tissue culture dish (GreinerBioOne) and cultured for 24 h to give 40-50% confluence. siRNA duplexes are transfected with DharmaFect (#4) reagent at a concentration of 25 nM. The cells are analysed 24-72 h after siRNA transfection.

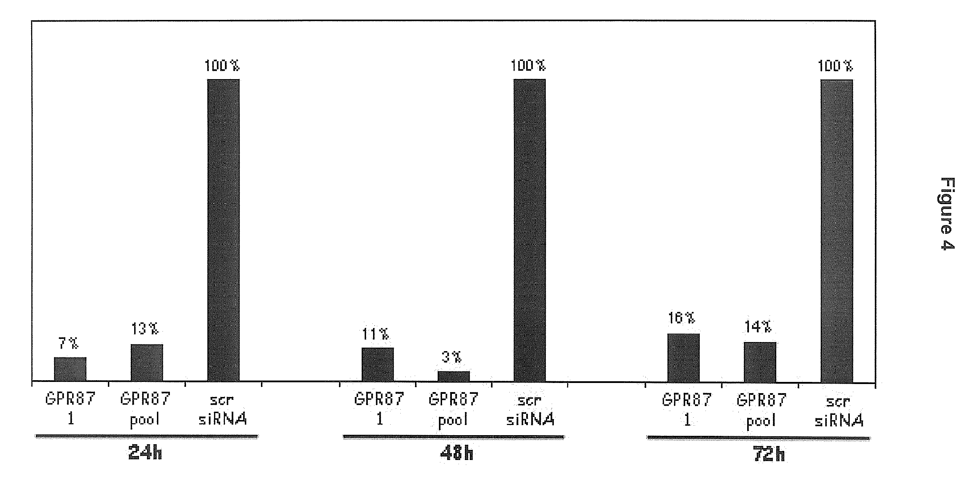

The knockdown as verified by quantitative PCR on mRNA level is shown in Fig. 4:
GPR87-1: siRNA (targeting sequence SEQ ID NO:3)
GPR87-pool: siRNA pool obtained from Dharmacon including siRNA (targeting sequence SEQ ID NO:3) and three other siRNAs targeting GPR87 mRNA
scr siRNA: siCONTROL #1 from Dharmacon

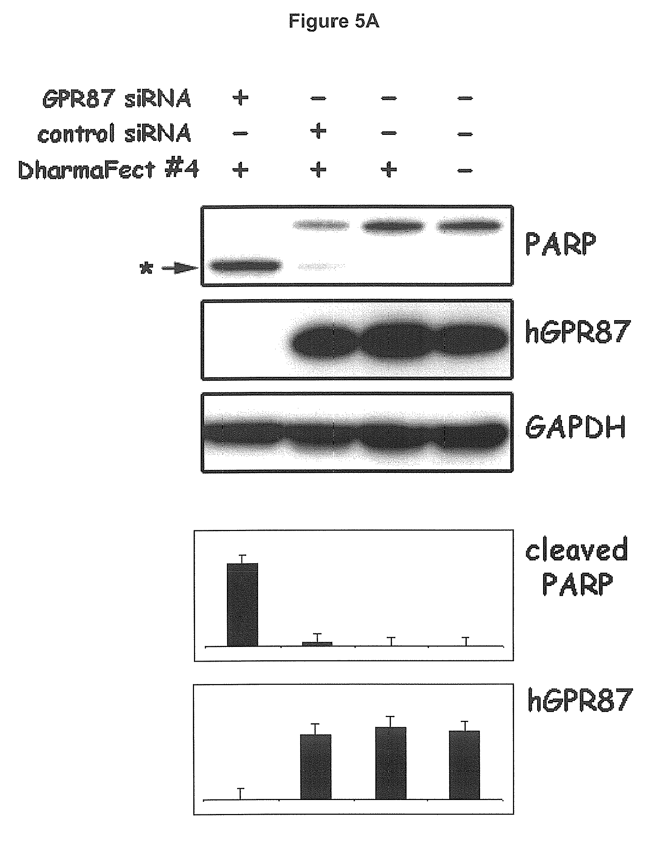

In addition, the knockdown is verified by Western blot analysis on protein level, using a GPR87 specific antibody (Abcam) recognizing the intracellular C-terminal domain of human GPR87. Fig. 5A shows PARP cleavage, hGPR87 knockdown, and relative quantification of PARP and hGPR87 respectively, with ImageQuant software package DharmaFect#4. DharmaFect transefction reagent #4 is used to transfect HN5 cells with siRNAs and is used alone as control for the effect of the transfection reagent.
hGPR87: Western blot analysis of hGPR87 protein.
GAPDH: loading control. The band marked with an asterisk indicates cleaved PARP.

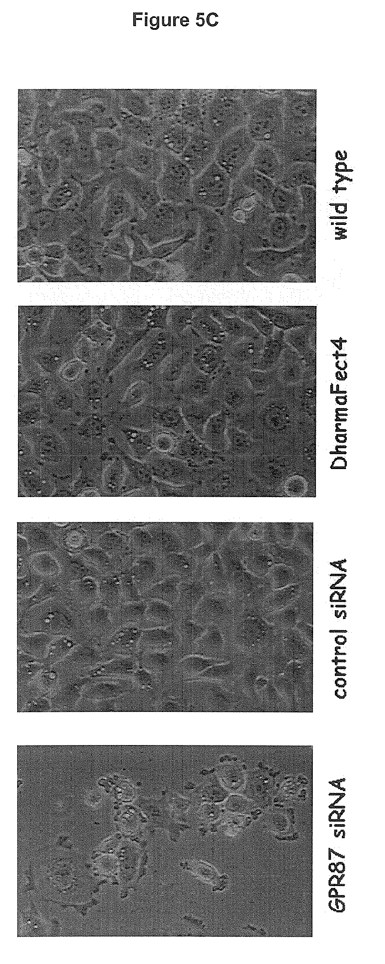

The phenotypic effects after siRNA treatment and down-regulation of human GPR87 protein are monitored by lightmicroscopy (Fig. 5C). The cells exhibit typical characteristics of apoptotic cells. The induction of apoptosis is measured by detection of cleaved PARP protein. PARP cleavage is dependent on the activation of caspases, and therefore is an indicator for early apoptosis. HN5 cells, which have lost human GPR87 protein upon siRNA treatment, show a significant increase in cleaved PARP protein (Fig. 5A) indicating the essential role of hGPR87 for survival of tumor cells.

The RT-PCR analysis of siRNA treated cells is performed as follows: siRNA treated cells are lysed 48 h after transfection by Trizol reagent. To harvest and lyse cells, the medium is aspirated and 500 µl Trizol reagent per well are added. The lysates are homogenized by incubating for 5 min at RT. 100 µl chloroform per 500 µl Trizol reagent is added and shaken intensively for 30 sec and additionally incubated for 3 min at RT. The two phases are separated again by a centrifugation step at 10000 rpm for 5 min at 4°C. The upper aqueous phase is transferred into a fresh Eppendorf tube. The RNA is precipitated with 250 µl 2-propanol per 500 µl Trizol prep for 15 min at 4°C, including a 15 min centrifugation step at 14000 rpm at 4°C. The supernatant is discarded and the RNA-pellet is washed with ice-cold 1 ml 75% EtOH and centrifuged at 14000 rpm for 5 min at 4°C. Finally the RNA-pellet is resuspended in 10 µl DEPC-H₂O and used immediately for single-strand-cDNA synthesis. For single-strand-cDNA synthesis an oligo dT primer is used in combination with the Superscript III polymerase. 9 µl of the RNA preparation are mixed with 1 µl 100 mM oligo dT primer and incubated at 70°C for 10 min as a pre-denaturing step. 10 µl of the given master mix (MM) are added and the reaction is incubated at 48°C for 60 min to synthesis the cDNA. Finally 30 µl of DEPC-H₂O are added to give a final volume of 50 µl. For RT-PCR analysis a hGPR87 specific Taqman-gene-expression assay (Applied Biosystems) is used as described by the manufacturer.

Western blot analysis (results shown in Fig. 5A) of the siRNA-treated cells is performed as follows: siRNA-treated cells for immunoblots are rinsed with cold phosphate-buffered saline (PBS, 17 mM KH₂PO₄, 50 mM Na₂HPO₄ and 150 mM NaCl) and scraped off. After centrifugation the cell pellet is resuspended in lysis buffer containing 20 mM Tris (pH 7.5), 5 mM EDTA, 2 mM EGTA, 0,5% Triton X-100, 30 mM sodium fluoride, 20 mM sodium pyrophosphate, 40 mM ß-glycerophosphate, 1 mM sodium orthovanadate, 10 uM leupeptin, 5 uM pepstatin A, 3 mM benzamidine and supplemented with 1 mM phenylmethylsulfonyl fluoride. For immunoblotting, 75 ug of the cell lysates are loaded onto precast 4-12% gradient Tris-glycine gels (BIO-RAD, CA, USA) and electrophoresed at 100 V. After transfer to Hybond nitrocellulose membranes (Amersham, Buckinghamshire, UK) at 170 mA at RT for 1 h, the blots are blocked in 5% dried milk in TBS-T (Tris-buffered-saline plus Tween) for 1 h at room temperature and probed o/n at 4°C with the primary antibodies. Antibodies for hGPR87 and GAPDH are from Abcam (Cambridge, UK), anti-PARP antibody is from cell signaling technologies (Danvers, USA/MA). Detection of the bound antibody is done by ECL (Amersham).

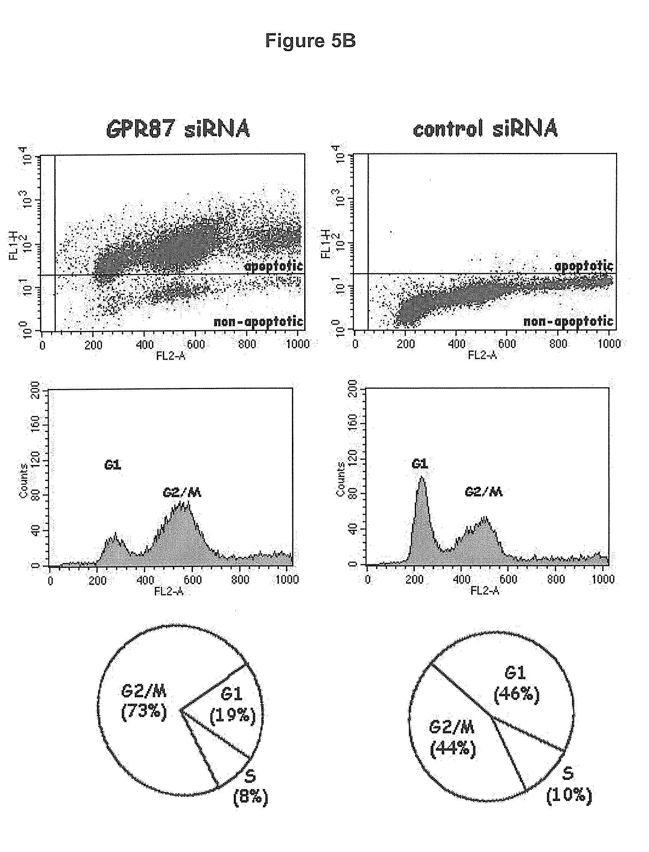

Another characteristic feature of apoptosis is the appearance of DNA double-strand breaks (DSBs), at the start of DNA fragmentation, which is a feature of late stage apoptosis. In hGPR87 siRNA-treated cells these DSBs can be detected by the ApoDirect kit, which labels DSBs with a fluorescent dye (Fig. 5B). The ApoDirect assay in siRNA-treated cells is performed as follows: siRNA-treated cells for ApoDirect analysis are rinsed with cold phosphate-buffered saline (PBS, 17 mM KH₂PO₄, 50 mM Na₂HPO₄ and 150 mM NaCl) and scraped off. The floating cells are collected by centrifugation and combined with the adherent cell fraction. After centrifugation, the cells are washed again in PBS and after the second centrifugation step the cell pellet is resuspended in 5 ml 1% paraformaldehyde and incubated on ice for 15 min. After fixation the cells are washed twice in PBS and then resuspended in ice-cold 70% ethanol and incubated on ice for 30 min. The cells are stored at -20°C o/n and stained the following day. For the detection of apoptotic cells and their cell cycle distribution the Apo-Direct kit from BD Pharmingen is used. The staining protocol is performed following manufacturer's instructions. The stained samples are subsequentially analyzed by FACS using a FACSCalibur device (BectonDickenson) and the CeliQuest software package.

In parallel, a propidium iodide staining is performed to analyze the cell cycle profile of the apoptotic HN5 cells after GPR87 knock-down. The cell cycle distribution of apoptotic cells indicates an essential impact of human GPR87 on the cell cycle progression (e.g. apoptotic cells accumulate in G1-phase; Fig. 5B). In general, it can be shown that the knock-down of human GPR87 protein has a major influence on viability, survival and cell cycle progression of head & neck squamous cell carcinoma cells.

### Example 4

### Inhibition of proliferation and induction of apoptosis with a specific antibody directed against the extracellular N-terminal part of hGPR87

It can be shown that not only depletion of the hGPR87 protein, but also blocking of hGPR87 by an antibody directed against the extracellular N-terminal domain has an essential impact on the viability and survival of a head and neck squamous cell carcinoma cell line HN5. The N-terminal domain of hGPR87 is located outside the cell membrane and therefore available for antibody targeting. To reduce unspecific effects the cells are treated with the indicated antibody in serum free conditions. HN5 cells can proliferate in serum free conditions for at least 6 days (data not shown).

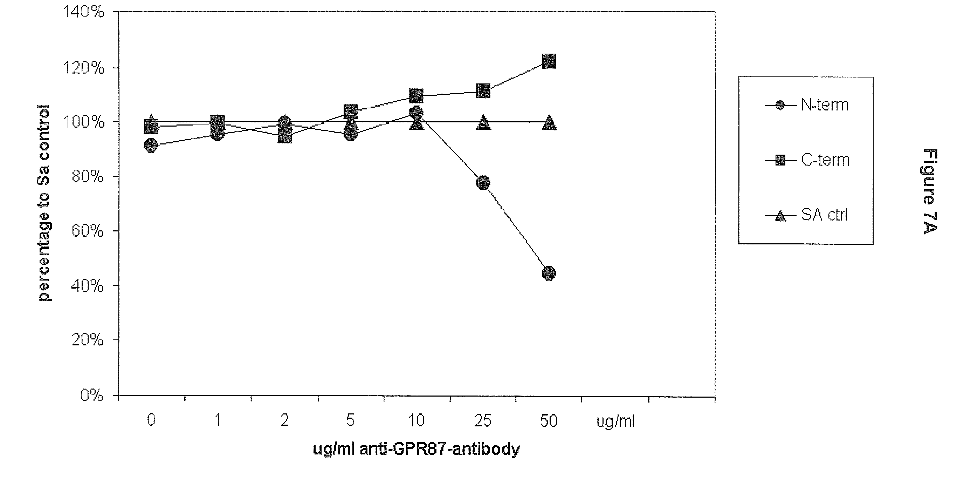

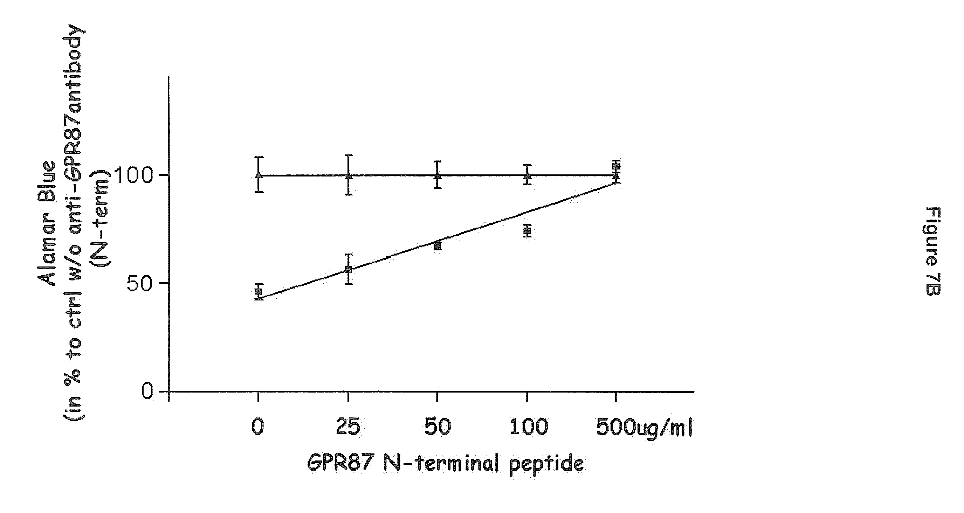

Antibody targeting of hGPR87 on living cells is performed as follows: For antibody applications on living HN5 cells, a rabbit anti-hGPR87, directed against the N-terminus of hGPR87 (Novus Biologicals) is used (in Fig. 7A: N-term). HN5 cells are seeded (75000/6-well and 3000/96-well) the day prior to the antibody treatment. The antibody is used at a concentration of 50 ug/ml in medium without serum. 48 h after antibody addition the cells are either lysed for western blot analysis or analysed by AlamarBlue staining. Briefly, 10 ul of AlamarBlue reagent (Biosource, Camarillo USA/CA) are added to 100 ul medium and incubated until a change in color can be recognized (~6 hours). As a control, 50ug/ml antibody targeting C-terminal domain of human GPR87 is used (in Fig. 7A: C-term). Since antibodies are dissolved in PBS containing 0.01 %sodium azide, 0.01% sodium azide in PBS is added to the cells as a control to exclude toxic side effects (SA ctrl in Fig. 7A).

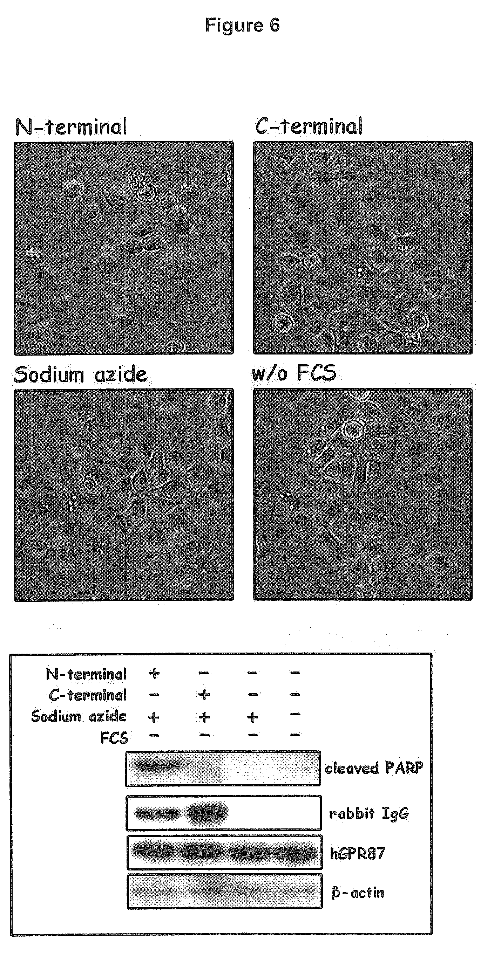

The proliferation rate of HN5 cells is dramatically reduced by adding the indicated antibody. This reduction in proliferation is accompanied by an increase in apoptotic cells, shown by light microscopy (Fig. 6, upper panel) and PARP-cleavage detection (Fig. 6, lower panel).

### Fig. 6, upper panel:

N-terminal: 50 ug/ml antibody targeting N-terminal domain of human GPR87 is added.
C-terminal: 50 ug/ml antibody targeting C-terminal domain of human GPR87 is added as a control.
Sodium azide: antibodies are dissolved in PBS containing 0.01% sodium azide, therefore 0.01 % sodium azide in PBS iss added to exclude toxic side effects.
w/o FCS: medium without fetal calf serum is used.

### Fig. 6, lower panel:

N-terminal: 50ug/ml antibody targeting N-terminal domain of human GPR87 is added to the cells.
C-terminal: 50ug/ml antibody targeting C-terminal domain of human GPR87 is added to the cells.
Sodium azide: antibodies are dissolved in PBS containing 0.01% sodium azide, therefore 0.01 % sodium azide in PBS is added to the cells.
w/o FCS: medium without fetal calf serum is added to the cells.

As can be seen from Fig. 7A, the anti-proliferative, pro-apoptotic effect is dependent on the antibody concentration.

To determine the specificity of the anti-GPR87 antibody recognizing the N-terminus, in a so-called "rescue experiment", a peptide covering the first 46 amino acids of SEQ ID NO:2, is added. (The amounts used are 50 ug/ml of antibody and increasing amounts of GPR87 N-terminal peptide). As can be seen from Fig. 7B, the pro-apoptotic, anti-proliferative effect can be reversed by pre-incubation of the antibody with the peptide. The reversibility of the effects (proliferation and viability) correlates with the concentration of the blocking peptide (Fig. 7B).

It has to be noted that inhibition of proliferation and induction of apoptosis in HN5 cells is merely due to the inactivation of hGPR87 (Fig. 6, lower panel) and not based on the downregulation of hGPR87 protein (as shown in Example 3 via the siRNA approach). This result clearly demonstrates that inhibition of hGPR87 (via sterical hindrance or direct neutralization) can already lead to inhibition of proliferation and induction of apoptosis in tumor cells. The data obtained in this experiment provide the proof of concept that using small molecule inhibitors of hGPR87 is a feasible approach to aim at a novel tumor therapy based on GPR87 inhibition.

### References

Abbracchio, M. P. et al. "Characterization of the UDP-glucose receptor (re-named here the P2Y14 receptor) adds diversity to the P2Y receptor family." Trends Pharmacol.Sci. 24.2 (2003): 52-55.
Aihara, H. and J. Miyazaki. "Gene transfer into muscle by electroporation in vivo." Nat.Biotechnol. 16.9 (1998): 867-70.
Allen, LF., Lefkowitz RJ, Caron MG, Cotecchia S. "G-protein-coupled receptor genes as protooncogenes: constitutively activating mutation of the alpha 1B-adrenergic receptor enhances mitogenesis and tumorigenicity." Proc Natl Acad Sci U S A. 1991, Dec 15;88(24): 11354-8.
Angers, S. et al. "Detection of beta 2-adrenergic receptor dimerization in living cells using bioluminescence resonance energy transfer (BRET)." Proc.Natl.Acad.Sci.U.S.A 97.7 (2000): 3684-89.
Arnon et al. (1985). Monoclonal Antibodies For Immunotargeting Of Drugs In Cancer Therapy. Monoclonal Antibodies And Cancer Therapy, Reisfeld et al. (eds.) pp. 243-56.
Aujame L., Geoffroy F., Sodoyer R. (1997). High affinity human antibodies by phage display. Hum Antibodies 8(4):155-68.
Baldwin et al. (1985). (eds.) pp. 303-16 (Academic Press).
Baron, U. and H. Bujard. "Tet repressor-based system for regulated gene expression in eukaryotic cells: principles and advances." Methods Enzymol. 327 (2000): 401-21.
Barry, M. A., M. E. Barry, and S. A. Johnston. "Production of monoclonal antibodies by genetic immunization." Biotechniques 16. 4 (1994): 616-8, 620.
Berger, J. et al. "Secreted placental alkaline phosphatase: a powerful new quantitative indicator of gene expression in eukaryotic cells." Gene 66.1 (1988): 1-10.
Bernstein, E. et al. "Role for a bidentate ribonuclease in the initiation step of RNA interference." Nature 409.6818 (2001): 363-66.
Boss, V., D. J. Talpade, and T. J. Murphy. "Induction of NFAT-mediated transcription by Gq-coupled receptors in lymphoid and non-lymphoid cells." J.Biol.Chem. 271.18 (1996): 10429-32.
Boulianne G. L., Hozumi N. and Shulman, M.J. (1984). Production of functional chimeric mouse/human antibody. Nature 312:643.
Brams Peter, Mai-Lan Nguyen, Soulaima Chamat, lvor Royston and Phillip R. Morrow. (1998) Antigen-Specific IgG Responses from Naive Human Splenocytes: In Vitro Priming Followed by Antigen Boost in the SCID Mouse.The Journal of Immunology, 160: 2051-2058.
Breitling F and Duebel S. Recombinant Antibodies. New York: John Wiley, 1999.
Brini, M. et al. "Transfected aequorin in the measurement of cytosolic Ca2+ concentration ([Ca2+]c). A critical evaluation." J.Biol.Chem. 270.17 (1995): 9896-903.
Bronstein, I. et al. "Chemiluminescence: sensitive detection technology for reporter gene assays." Clin.Chem. 42.9 (1996): 1542-46.
Burnstock, G. and C. Kennedy. "Is there a basis for distinguishing two types of P2-purinoceptor?" Gen.Pharmacol. 16.5 (1985): 433-40.
Cacace, A. et al. "An ultra-HTS process for the identification of small molecule modulators of orphan G-protein-coupled receptors." Drug Discov.Today 8.17 (2003): 785-92.
Chin, J. et al. "Miniaturization of cell-based beta-lactamase-dependent FRET assays to ultra-high throughput formats to identify agonists of human liver X receptors." Assay.Drug Dev.Technol. 1.6 (2003): 777-87.
Chothia and Lesk. (1987). J. Mol. Biol. 196: 901-917.
Colby DW, Kellogg BA, Graff CP, Yeung YA, Swers JS, Wittrup KD. (2004). "Engineering antibody affinity by yeast surface display." Methods Enzymol. 388:348-58.
Coligan, et al., Current Protocols in Immunology (2): Chapter 5 (1991).
Coward, P. et al. "Chimeric G proteins allow a high-throughput signaling assay of Gi-coupled receptors." Anal.Biochem. 270.2 (1999): 242-48.
Cubitt, A. B. et al. "Understanding, improving and using green fluorescent proteins." Trends Biochem.Sci. 20.11 (1995): 448-55.
Daugherty PS, Chen G, Olsen MJ, Iverson BL, Georgiou G. (Sep. 1998). Antibody affinity maturation using bacterial surface display. Protein Eng. 11(9):825-32.
de Wet, J. R. et al. "Firefly luciferase gene: structure and expression in mammalian cells." Mol.Cell Biol. 7.2 (1987): 725-37.
Dolznig, H. et al. "Characterization of cancer stroma markers: in silico analysis of an mRNA expression database for fibroblast activation protein and endosialin." Cancer Immun. 5 (2005): 10.
Doronina SO, Toki BE, Torgov MY, Mendelsohn BA, Cerveny CG, Chace DF, DeBlanc RL, Gearing RP, Bovee TD, Siegall CB, Francisco JA, Wahl AF, Meyer DL, Senter PD. (2003). Development of potent monoclonal antibody auristatin conjugates for cancer therapy. Nat Biotechnol. Jul. 21 (7):778-84. Epub 2003 Jun 1.
Eccles, S. A. "Monoclonal antibodies targeting cancer: 'magic bullets' or just the trigger?" Breast Cancer Res. 3.2 (2001): 86-90.
Figini et al. (1998). Panning phage antibody libraries on cells: isolation of human Fab fragments against ovarian carcinoma using guided selection. Cancer Res. 58:991-6.
Fire, A. et al. "Potent and specific genetic interference by double-stranded RNA in Caenorhabditis elegans." Nature 391. 6669 (1998): 806-11.
Frank et al. (1987). Methods Enzymol. 154:221-249.
Fredholm, B. B. et al. "Towards a revised nomenclature for P1 and P2 receptors." Trends Pharmacol.Sci. 18.3 (1997): 79-82.
Gait M.J. (1984). Oligonucleotide Synthesis. A Practical Approach. IRL Press, Oxford, UK.
Gee, K. R. et al. "Chemical and physiological characterization of fluo-4 Ca(2+)-indicator dyes." Cell Calcium 27.2 (2000): 97-106.
George, S. E., P. J. Bungay, and L. H. Naylor. "Functional coupling of endogenous serotonin (5-HT1 B) and calcitonin (C1 a) receptors in CHO cells to a cyclic AMP-responsive luciferase reporter gene." J. Neurochem. 69.3 (1997): 1278-85.
Ghosh, RN., Chen, YT., DeBiasio, R., DeBiasio, RL., Conway, BR., Minor, LK., and Demarest,KT. (2000). "Cell-based, high-content screen for receptor internalization, recycling and intracellular trafficking." Biotechniques 29, 170-175.
Ghosh, RN., DeBiasio, R., Hudson, CC., Ramer, ER., Cowan, CL., and Oakley, RH. (2005). "Quantitative cell-based high-content screening for vasopressin receptor agonists using transfluor technology." J Biomol Screen. 10, 476-484.
Gonzalez, F. A. et al. "Activation of early events of the mitogenic response by a P2Y purinoceptor with covalently bound 3'-O-(4-benzoyl)-benzoyladenosine 5'-triphosphate." Proc.Natl.Acad.Sci.U.S.A 87.24 (1990): 9717-21.
Gopalakrishnan, S. M. et al. "A cell-based microarrayed compound screening format for identifying agonists of G-protein-coupled receptors." Anal.Biochem. 321.2 (2003): 192-201.
Granas, C. et al. "High content screening for G protein-coupled receptors using cell-based protein translocation assays." Comb.Chem.High Throughput.Screen. 8.4 (2005): 301-09.
Gussow, D. and G. Seemann. "Humanization of monoclonal antibodies." Methods Enzymol. 203 (1991): 99-121.
Gutkind, J. S. "Cell growth control by G protein-coupled receptors: from signal transduction to signal integration." Oncogene 17. 11 Reviews (1998): 1331-42.
Harlow L.D. "Antibodies." Cold Spring Harbor Lab, 1988.
Hayden and Mandecki. (1988). Gene synthesis by serial cloning of oligonucleotides. DNA 7(8): 571-7.
He Mingyue, and Taussig Michael J. (1 July 2002). Ribosome display: cell-free protein display technology. Briefings in Functional Genomics and Proteomics, Volume 1, Number 2, pp. 204-212(9).
Heasley, L. E. "Autocrine and paracrine signaling through neuropeptide receptors in human cancer." Oncogene 20.13 (2001): 1563-69.
Hellstrom et al. (1987). Antibodies For Drug Delivery. Controlled Drug Delivery (2nd Ed.), Robinson et al. (eds.), pp. 623-53 (Marcel Dekker, Inc.).
Hepler, J. R. and A. G. Gilman. "G proteins." Trends Biochem.Sci. 17.10 (1992): 383-87.
Himmler, A., C. Stratowa, and A. P. Czernilofsky. "Functional testing of human dopamine D1 and D5 receptors expressed in stable cAMP-responsive luciferase reporter cell lines." J.Recept.Res. 13.1-4 (1993): 79-94.
Hopkins, A. L. and C. R. Groom. "The druggable genome." Nat.Rev.Drug Discov. 1.9 (2002): 727-30.
Hudson PJ, and Souriau C. (2001 Sep.). Expert Opin Biol Ther. 1 (5): 845-55.
Jain, V. K. and I. T. Magrath. "A chemiluminescent assay for quantitation of beta-galactosidase in the femtogram range: application to quantitation of beta-galactosidase in lacZ-transfected cells." Anal.Biochem. 199.1 (1991): 119-24.
Jerman, J. C. et al. "Pharmacological characterisation of human 5-HT2 receptor subtypes." Eur.J.Pharmacol. 414.1 (2001): 23-30.
Jespers et al. (1994). Bio/Technology 12:899-903.
Johnson, S. and R. E. Bird. "Construction of single-chain Fv derivatives monoclonal antibodies and their production in Escherichia coli." Methods Enzymol. 203 (1991): 88-98.
Joost, P. and A. Methner. "Phylogenetic analysis of 277 human G-protein-coupled receptors as a tool for the prediction of orphan receptor ligands." Genome Biol. 3.11 (2002): RESEARCH 0063.
Kabat E. A., Wu T. T., Perry H. M., Gottesman K. S. and Foeller C. (1991). Sequences of Proteins of Immunological Interest (5th Ed.). NIH Publication No. 91-3242. U.S. Department of Health and Human Services, Public Health Service, National Institutes of Health, Bethesda, MD.
Kim, S. G. et al. "Tumor necrosis factor alpha-induced apoptosis in astrocytes is prevented by the activation of P2Y6, but not P2Y4 nucleotide receptors." Biochem.Pharmacol. 65.6 (2003): 923-31.
King David M. et al., (2004). Phase I Clinical Trial of the Immunocytokine EMD 273063 in Melanoma Patients. J Clin Oncol 22: 4463-4473.
Kontermann Roland E. (Jan. 2005). Recombinant bispecific antibodies for cancer therapy. Acta Pharmacologica Sinica 26 (1): 1-9.
Kornienko, O. et al. "Miniaturization of whole live cell-based GPCR assays using microdispensing and detection systems." J. Biomol. Screen. 9.3 (2004): 186-95.
Kreitman, R. J. et al. "Pseudomonas exotoxin-based immunotoxins containing the antibody LL2 or LL2-Fab' induce regression of subcutaneous human B-cell lymphoma in mice." Cancer Res. 53.4 (1993): 819-25.
Labrousse, H. et al. "Miniaturization of beta-galactosidase immunoassays using chromogenic and fluorogenic substrates." J.Immunol.Methods 48.2 (1982): 133-47.
Lazar, Greg A., et al., 2006, "Engineered antibody Fc variants with enhanced effector function", PNAS, March 14, 2006, Vol. 103, No. 11: 4005-4010
Le Poul, E. et al. "Adaptation of aequorin functional assay to high throughput screening." J.Biomol.Screen. 7.1 (2002): 57-65.
Li, S., S. Huang, and S. B. Peng. "Overexpression of G protein-coupled receptors in cancer cells: involvement in tumor progression." Int.J.Oncol. 27.5 (2005): 1329-39.
Lonberg and Huszar. (1995). Int. Rev. Immunol. 13:65-93.
Marinissen, M. J. and J. S. Gutkind. "G-protein-coupled receptors and signaling networks: emerging paradigms." Trends Pharmacol.Sci. 22.7 (2001): 368-76.
McCafferty et al. (1990). Nature, 348: 552-554.
Metaye, T. et al. "Pathophysiological roles of G-protein-coupled receptor kinases." Cell Signal. 17.8 (2005): 917-28.
Michelini, E. et al. "Development of a bioluminescence resonance energy-transfer assay for estrogen-like compound in vivo monitoring." Anal.Chem. 76.23 (2004): 7069-76.
Montminy, M. R., G. A. Gonzalez, and K. K. Yamamoto. "Regulation of cAMP-inducible genes by CREB." Trends Neurosci. 13.5 (1990): 184-88.
Moore, J. T., S. T. Davis, and I. K. Dev. "The development of beta-lactamase as a highly versatile genetic reporter for eukaryotic cells." Anal. Biochem. 247.2 (1997): 203-09.
Nayak BP, Tuteja R, Manivel V, Roy RP, Vishwakarma RA, Rao KV. (1998 Oct. 1). B cell responses to a peptide epitope. V. Kinetic regulation of repertoire discrimination and antibody optimization for epitope. J Immunol. 161 (7):3510-9.
Naylor, L. H. "Reporter gene technology: the future looks bright." Biochem.Pharmacol. 58.5 (1999): 749-57.
Newton Dianne L. and Rybak Susanna M. (2001). Antibody targeted therapeutics for lymphoma: new focus on the CD22 antigen and RNA. Expert Opin. Biol. Ther. 1(6):995-1003.
Niwa Rinpei, Mikiko Sakurada, Yukari Kobayashi, Aya Uehara, Kouji Matsushima, Ryuzo Ueda, Kazuyasu Nakamura, and Kenya Shitara. (March 15, 2005). "Dependent Cellular Cytotoxicity Induction at Lower Antigen Density." Vol. 11, 2327-2336, Clinical Cancer Research 2327.
Parma, J. et al. "Somatic mutations in the thyrotropin receptor gene cause hyperfunctioning thyroid adenomas." Nature 365.6447 (1993): 649-51.
Pfleger, K. D. and K. A. Eidne. "New technologies: bioluminescence resonance energy transfer (BRET) for the detection of real time interactions involving G-protein coupled receptors." Pituitary. 6.3 (2003): 141-51.
Pinchera et al. (1985). Analysis, Results, And Future Prospective Of The Therapeutic Use Of Radiolabeled Antibody In Cancer Therapy. Monoclonal Antibodies For Cancer Detection And Therapy (eds.) pp. 475-506.
Revets H, De Baetselier P, Muyldermans S. (2005 Jan.). Nanobodies as novel agents for cancer therapy. Expert Opin Biol Ther. 5(1):111-24.
Sambrook. 2 ed. Cold Spring Habor, N.Y.: Cold Spring Habor Laboratory Press, 1989.
Schiller, H. et al. "Solubilization and purification of the human ETB endothelin receptor produced by high-level fermentation in Pichia pastoris." Receptors.Channels 7.6 (2001): 453-69.
Schindler, M. and Doods, HN. (2002). "Binding properties of the novel, non-peptide CGRP receptor antagonist radioligand, [(3)H]BIBN4096BS." Eur J Pharmacol. 442, 187-193
Schnitzer, R. and Sommergruber, W. (2006) "The use of genetically engineered cell-based assays in in-vitro drug discovery." in "New Approaches to theGeneration and Evaluation of Chemical Diversity", in "Exploiting Chemical Diversity for Drug Discovery" Paul A. Bartlett and Michael Entzeroth, RSC Publishing, Royal Society of Chemistry, 2006.
Schoneberg, T. et al. "Mutant G-protein-coupled receptors as a cause of human diseases." Pharmacol.Ther. 104.3 (2004): 173-206.
Seifert, R. et al. "Examining the efficiency of receptor/G-protein coupling with a cleavable beta2-adrenoceptor-gsalpha fusion protein." Eur.J.Biochem. 260.3 (1999a): 661-66.
Seifert, R., K. Wenzel-Seifert, and B. K. Kobilka. "GPCR-Galpha fusion proteins: molecular analysis of receptor-G-protein coupling." Trends Pharmacol.Sci. 20.9 (1999b): 383-89.
Shen-Ong, G. L., Y. Feng, and D. A. Troyer. "Expression profiling identifies a novel alpha-methylacyl-CoA racemase exon with fumarate hydratase homology." Cancer Res. 63.12 (2003): 3296-301.
Shields, Robert L., et al., 2001, "High Resolution Mapping of the Binding Site on Human IgG1 for FcγRI, FcγRII, FcγRIII, and FcRn and Design of IgG1 Variants with Improved Binding to the FcγR*." J Biol. Chem., Vol. 276, No. 9, Issue of March 2, pp. 6591-6604, 2001.
Shin, S. U. and S. L. Morrison. "Production and properties of chimeric antibody molecules." Methods Enzymol. 178 (1989): 459-76.
Srinivasan, M., and Roeske, RW., 2005, "Immunomodulatory peptides from IgSF proteins: a review." Curr Protein Pept Sci., 2005, Apr; 6(2):185-96.
Stables, J. et al. "A bioluminescent assay for agonist activity at potentially any G-protein-coupled receptor." Anal.Biochem. 252.1 (1997): 115-26.
Stemmer et al. (1995). Single-step assembly of a gene and entire plasmid from large numbers of oligodeoxyribonucleotides, Gene 164(1): 49-53.
Stolz, U. et al. "Darwinian natural selection for orange bioluminescent color in a Jamaican click beetle." Proc.Natl.Acad.Sci.U.S.A 100.25 (2003): 14955-59.
Stratowa, C. and M. Audette. "Transcriptional regulation of the human intercellular adhesion molecule-1 gene: a short overview." Immunobiology 193.2-4 (1995): 293-304.
Suto C.and Ignar D. "Selection of an Optimal Reporter Gene for Cell-Based High Throughput Screening Assays." Journal of Biomolecular Screening 2 (1997): 7-9.
Tanha J, Xu P, Chen Z, Ni F, Kaplan H, Narang SA, MacKenzie CR. (2001 Jul. 6). Optimal design features of camelized human single-domain antibody libraries. J Biol Chem. 276(27): 24774-80. Epub 2001 May 2.
Thorpe et al. (1982). The Preparation And Cytotoxic Properties Of Antibody-Toxin Conjugates. Immunol. Rev., 62:119-58.
Thorpe. (1985). Antibody Carriers Of Cytotoxic Agents In Cancer Therapy: A Review. Monoclonal Antibodies 84: Biological And Clinical Applications. Pinchera et al. (eds.) pp. 475-506.
Tsai, V. et al. "In vitro immunization and expansion of antigen-specific cytotoxic T lymphocytes for adoptive immunotherapy using peptide-pulsed dendritic cells." Crit Rev.lmmunol. 18. 1-2 (1998): 65-75.
Tsien, R. Y. "The green fluorescent protein." Annu.Rev.Biochem. 67 (1998): 509-44.
Tu, M. T. et al. "P2Y(2) receptor-mediated proliferation of C(6) glioma cells via activation of Ras/Raf/MEK/MAPK pathway." Br.J.Pharmacol. 129.7 (2000): 1481-89.
Urlaub G. and Chasin LA. (1980). Isolation of Chinese hamster cell mutants deficient in dihydrofolate reductase activity. Proc Natl Acad Sci U S A. 1980 Jul;77(7):4216-20
Urlaub G. et al. (1983). Cell, Jun;33(2):405-412.
Vilardaga, J. P. et al. "Measurement of the millisecond activation switch of G protein-coupled receptors in living cells." Nat.Biotechnol. 21.7 (2003): 807-12.
Voraberger, G., R. Schafer, and C. Stratowa. "Cloning of the human gene for intercellular adhesion molecule 1 and analysis of its 5'-regulatory region. Induction by cytokines and phorbol ester." J. Immunol. 147.8 (1991): 2777-86.
Vrecl, M. et al. "Development of a BRET2 screening assay using beta-arrestin 2 mutants." J.Biomol.Screen. 9.4 (2004): 322-33.
Weyer, U. et al. "Establishment of a cellular assay system for G protein-linked receptors: coupling of human NK2 and 5-HT2 receptors to phospholipase C activates a luciferase reporter gene." Receptors.Channels 1.3 (1993): 193-200.
Widera, G. et al. "Increased DNA vaccine delivery and immunogenicity by electroporation in vivo." J. Immunol. 164.9 (2000): 4635-40.
Winter, G. et al. "Making antibodies by phage display technology." Annu.Rev.lmmunol. 12 (1994): 433-55.
Wolff, J. A. et al. "Direct gene transfer into mouse muscle in vivo." Science 247.4949 Pt 1 (1990): 1465-68.
Wood, K. V. et al. "Complementary DNA coding click beetle luciferases can elicit bioluminescence of different colors." Science 244.4905 (1989): 700-02.
Ye et al. (1992). "Gene synthesis and expression in E. coli for pump, a human matrix metalloproteinase." Biochem Biophys Res Commun 186(1):143-9.
Young, D. et al. "Isolation and characterization of a new cellular oncogene encoding a protein with multiple potential transmembrane domains." Cell 45.5 (1986): 711-19.
Yu, J. Y., S. L. DeRuiter, and D. L. Turner. "RNA interference by expression of short-interfering RNAs and hairpin RNAs in mammalian cells." Proc.Natl.Acad.Sci.U.S.A 99.9 (2002): 6047-52.
Zapata et al. (1995). Protein Eng. 8(10): 1057-1062.
Zeder-Lutz, G. et al. "Dot-blot immunodetection as a versatile and high-throughput assay to evaluate recombinant GPCRs produced in the yeast Pichia pastoris." Protein Expr.Purif. (2006, Jun. 8).

## Claims

1. The use of
a) a human GPR87 DNA molecule of SEQ ID NO: 1 or a variant or fragment thereof or a complement thereto, or of
b) a human GPR87 polypeptide of SEQ ID NO:2 or a variant or fragment thereof,
for the generation of an inhibitor of the biological function of human GPR87.

2. The use of claim 1, wherein said GPR87 molecule defined in a) or b) is used as an immunogen and the inhibitor is an anti-GPR87 antibody or an anti-GPR87 antibody fragment.

3. The use of claim 2, wherein the GPR87 molecule is a fragment from the N-terminus of the human GPR87 polypeptide or a DNA molecule encoding it.

4. The use of claim 3, wherein the fragment is contained within a peptide in the N-terminal extracellular domain of human GPR87, said peptide encompassing amino acids 1 to 46 of SEQ ID NO:2.

5. The use of claim 1, wherein a GPR87 molecule defined in a) is used and the inhibitor is an antisense oligonucleotide molecule, a small interfering RNA molecule or a ribozyme.

6. An antibody or an antibody fragment that specifically binds to one or more extracellular domains of human GPR87 of SEQ ID NO:2 for the treatment of cancer.

7. The antibody or antibody fragment of claim 6, which specifically binds to the N-terminus of human GPR87 of SEQ ID NO:2.

8. The antibody or antibody fragment of claim 7, which specifically binds to an epitope contained within a peptide encompassing amino acids 1 to 46 of SEQ ID NO:2.

9. An anti-GPR87 antibody of claim 6, which is polyclonal.

10. An anti-GPR87 antibody (fragment) of claim 6, which is monoclonal.

11. An anti-GPR87 antibody (fragment) of claim 6, which is recombinant.

12. An anti-GPR87 antibody (fragment) of claim 6, which is chimeric.

13. An anti-GPR87 antibody (fragment) of claim 6, which is humanized.

14. An anti-GPR87 antibody (fragment) of claim 6, which is fully human.

15. An antibody fragment of claim 6, which is a Fab fragment.

16. An anti-GPR87 antibody (fragment) that is conjugated to a therapeutic moiety or a radioactive metal ion.

17. An antisense oligonucleotide molecule that inhibits expression of GPR87.

18. A ribozyme that inhibits expression of GPR87.

19. A short interfering RNA (siRNA) molecule that inhibits expression of GPR87 by RNA interference.

20. An inhibitor of the biological function of human GPR87 as defined in any one of claims 17 to 19 for the treatment of cancer.

21. An GPR87 inhibitor as defined in any one of claims 6 to 19 for the treatment of a squamous cell carcinoma or metastases derived therefrom.

22. A pharmaceutical composition for the treatment of cancer, containing, as the active ingredient, an inhibitor of human GPR87 as defined in any one of claims 6 to 19.

23. A method for determining whether a test compound has the ability of modulating GPR87 activity by
a) binding to and activating GPR87 and/or a GPR87-G-alpha fusion polypeptide, and/or
b) inhibiting the generation of intracellular signals generated upon binding of endogenous ligands and thus activation of GPR87 by
i. binding to the GPR87 binding site of the endogenous ligand, or by
ii. allosteric hinderance of GPR87, or
c) inhibiting the interaction of GPR87 with its endogenous receptor ligands or
d) acting intracellularly on a signal in the signal cascade transduced upon activation of GPR87 upon ligand binding;
wherein cells expressing on their surface human GPR87 and/or a GPR87-G-alpha fusion polypeptide, or a membrane fraction of such cells, are incubated with said test compound and said test compound's ability to modulate GPR87 activity as defined in a) to d) is determined.

24. The method of claim 23, wherein said GPR87 is human GPR87 of SEQ ID NO:2.

25. The method of claim 24, wherein said cells are transfected to express a human GPR87-G-alpha fusion polypeptide, wherein the C-terminus of human GPR87 is fused to the N-terminus of a G-alpha subunit.

26. The method of claim 25, wherein said G-alpha subunit is selected from GNAQ, GNASL, GNASS and GNA15.

27. The method of claim 23, wherein said test compound is tested for its ability to inhibit binding of the endogenous ligand to GPR87.

28. The method of claim 23, wherein said test compound's ability to modulate hGR87 activity is determined by measuring a change in intracellular calcium concentration.

29. The method of claim 23, wherein said test compound's ability to modulate human GPR87 is determined by measuring change in activity of a reporter gene product in test cells that are genetically engineered to express a reporter molecule under the control of a regulatory sequence containing a promoter and one or more response elements that respond to a signal generated upon GPR87 activation.

30. The method of claim 29, wherein said response elements are responsive to a change in the concentration of inositol-1,4,5-triphosphate (IP3) and diacylglycerol (DAG).

31. The method of claim 29 or 30, wherein said reporter gene product is luciferase and wherein said response element is a cAMP responsive element.

32. The method of claim 23, wherein said test compound's ability to modulate GPR87 activity is determined by measuring its effect on conformational changes of human GPR87 upon activation.

33. The method of claim 23, wherein said test compound's ability to modulate human GPR87 activity is determined by measuring its effect on a signal generated upon the interaction of human GPR87 with beta-arrestin.

34. The method of claim 23, wherein said test compound's ability to modulate human GPR87 activity is determined by measuring its effect on translocation of a transcription factor from the cytoplasm to the nucleus upon human GPR87 activation.

35. The method of any one of claims 23 to 34, wherein, in addition, said test compound is testd for its effect on apoptosis and/or proliferation of tumor cells.

36. A human GPR87-G-alpha fusion polypeptide, wherein the C-terminus of human GPR87 is fused to the N-terminus of a G-alpha subunit.

37. The fusion polypeptide of claim 36, wherein said G-alpha subunit is selected from GNAQ, GNASL, GNASS and GNA15.


SEQUENCE LISTING

<110>  Boehringer Ingelheim International GmbH

<120>  Inhibitors of GPR87 for cancer therapy

<130>  12-0265

<160>  3

<170>  PatentIn version 3.3

<210>  1
<211>  1498
<212>  DNA
<213>  Homo sapiens

<220>
<221>  5'UTR
<222>  (1)..(361)

<220>
<221>  CDS
<222>  (362)..(1438)

<220>
<221>  3'UTR
<222>  (1439)..(1498)

<400>  1
ttacattagc aagagagcaa gttgttccag tagttgcctg gcaggagaat ttgaaagggt      60

gccccaaagg acaatctcta aaggggtaag ggagatacct accttgtctg gtaggggaga     120

tgtttcgttt tcatgcttta ccagaaaatc cacttccctg ctgaccttag tttcaaagct     180

tattcttaat tagagacaag aaacctgttt caacttgaag acaccgtatg aggtgaatgg     240

acagccagcc accacaatga aagaaatcaa accaggaata acctatgctg aacccacgcc     300

tcaatcgtcc ccaagtgttt cctgacacgc atctttgctt acagtgcatc acaactgaag     360

a atg ggg ttc aac ttg acg ctt gca aaa tta cca aat aac gag ctg cac      409
  Met Gly Phe Asn Leu Thr Leu Ala Lys Leu Pro Asn Asn Glu Leu His
  1               5                   10                  15

ggc caa gag agt cac aat tca ggc aac agg agc gac ggg cca gga aag      457
Gly Gln Glu Ser His Asn Ser Gly Asn Arg Ser Asp Gly Pro Gly Lys
            20                  25                  30

aac acc acc ctt cac aat gaa ttt gac aca att gtc ttg ccg gtg ctt      505
Asn Thr Thr Leu His Asn Glu Phe Asp Thr Ile Val Leu Pro Val Leu
        35                  40                  45

tat ctc att ata ttt gtg gca agc atc ttg ctg aat ggt tta gca gtg 553
Tyr Leu Ile Ile Phe Val Ala Ser Ile Leu Leu Asn Gly Leu Ala Val
    50                  55                  60

tgg atc ttc ttc cac att agg aat aaa acc agc ttc ata ttc tat ctc 601
Trp Ile Phe Phe His Ile Arg Asn Lys Thr Ser Phe Ile Phe Tyr Leu
65                  70                  75                  80

aaa aac ata gtg gtt gca gac ctc ata atg acg ctg aca ttt cca ttt 649
Lys Asn Ile Val Val Ala Asp Leu Ile Met Thr Leu Thr Phe Pro Phe
                85                  90                  95

cga ata gtc cat gat gca gga ttt gga cct tgg tac ttc aag ttt att 697
Arg Ile Val His Asp Ala Gly Phe Gly Pro Trp Tyr Phe Lys Phe Ile
            100                 105                 110

ctc tgc aga tac act tca gtt ttg ttt tat gca aac atg tat act tcc 745
Leu Cys Arg Tyr Thr Ser Val Leu Phe Tyr Ala Asn Met Tyr Thr Ser
        115                 120                 125

atc gtg ttc ctt ggg ctg ata agc att gat cgc tat ctg aag gtg gtc 793
Ile Val Phe Leu Gly Leu Ile Ser Ile Asp Arg Tyr Leu Lys Val Val
    130                 135                 140

aag cca ttt ggg gac tct cgg atg tac aac ata acc ttc acg aag gtt 841
Lys Pro Phe Gly Asp Ser Arg Met Tyr Asn Ile Thr Phe Thr Lys Val
145                 150                 155                 160

tta tct gtt tgt gtt tgg gtg atc atg gct gtt ttg tct ttg cca aac 889
Leu Ser Val Cys Val Trp Val Ile Met Ala Val Leu Ser Leu Pro Asn
                165                 170                 175

atc atc ctg aca aat ggt cag cca aca gag gac aat atc cat gac tgc 937
Ile Ile Leu Thr Asn Gly Gln Pro Thr Glu Asp Asn Ile His Asp Cys
            180                 185                 190

tca aaa ctt aaa agt cct ttg ggg gtc aaa tgg cat acg gca gtc acc 985
Ser Lys Leu Lys Ser Pro Leu Gly Val Lys Trp His Thr Ala Val Thr
        195                 200                 205

tat gtg aac agc tgc ttg ttt gtg gcc gtg ctg gtg att ctg atc gga 1033
Tyr Val Asn Ser Cys Leu Phe Val Ala Val Leu Val Ile Leu Ile Gly
    210                 215                 220

tgt tac ata gcc ata tcc agg tac atc cac aaa tcc agc agg caa ttc 1081
Cys Tyr Ile Ala Ile Ser Arg Tyr Ile His Lys Ser Ser Arg Gln Phe
225                 230                 235                 240

ata agt cag tca agc cga aag cga aaa cat aac cag agc atc agg gtt 1129
Ile Ser Gln Ser Ser Arg Lys Arg Lys His Asn Gln Ser Ile Arg Val
                245                 250                 255

gtt gtg gct gtg ttt ttt acc tgc ttt cta cca tat cac ttg tgc aga 1177
Val Val Ala Val Phe Phe Thr Cys Phe Leu Pro Tyr His Leu Cys Arg
            260                 265                 270

att cct ttt act ttt agt cac tta gac agg ctt tta gat gaa tct gca     1225
Ile Pro Phe Thr Phe Ser His Leu Asp Arg Leu Leu Asp Glu Ser Ala
        275                 280                 285

caa aaa atc cta tat tac tgc aaa gaa att aca ctt ttc ttg tct gcg     1273
Gln Lys Ile Leu Tyr Tyr Cys Lys Glu Ile Thr Leu Phe Leu Ser Ala
    290                 295                 300

tgt aat gtt tgc ctg gat cca ata att tac ttt ttc atg tgt agg tca     1321
Cys Asn Val Cys Leu Asp Pro Ile Ile Tyr Phe Phe Met Cys Arg Ser
305                 310                 315                 320

ttt tca aga agg ctg ttc aaa aaa tca aat atc aga acc agg agt gaa     1369
Phe Ser Arg Arg Leu Phe Lys Lys Ser Asn Ile Arg Thr Arg Ser Glu
                325                 330                 335

agc atc aga tca ctg caa agt gtg aga aga tcg gaa gtt cgc ata tat     1417
Ser Ile Arg Ser Leu Gln Ser Val Arg Arg Ser Glu Val Arg Ile Tyr
            340                 345                 350

tat gat tat act gat gtg tag gccttttatt gtttgttgga atcgatatgt        1468
Tyr Asp Tyr Thr Asp Val
        355

acaaagtgta aataaatgtt tcttttcatt                                    1498

<210>  2
<211>  358
<212>  PRT
<213>  Homo sapiens

<400>  2

Met Gly Phe Asn Leu Thr Leu Ala Lys Leu Pro Asn Asn Glu Leu His
1               5                   10                  15

Gly Gln Glu Ser His Asn Ser Gly Asn Arg Ser Asp Gly Pro Gly Lys
            20                  25                  30

Asn Thr Thr Leu His Asn Glu Phe Asp Thr Ile Val Leu Pro Val Leu
        35                  40                  45

Tyr Leu Ile Ile Phe Val Ala Ser Ile Leu Leu Asn Gly Leu Ala Val
    50                  55                  60

Trp Ile Phe Phe His Ile Arg Asn Lys Thr Ser Phe Ile Phe Tyr Leu
65                  70                  75                  80

Lys Asn Ile Val Val Ala Asp Leu Ile Met Thr Leu Thr Phe Pro Phe
                85                  90                  95

Arg Ile Val His Asp Ala Gly Phe Gly Pro Trp Tyr Phe Lys Phe Ile
            100                 105                 110

Leu Cys Arg Tyr Thr Ser Val Leu Phe Tyr Ala Asn Met Tyr Thr Ser
        115                 120                 125

Ile Val Phe Leu Gly Leu Ile Ser Ile Asp Arg Tyr Leu Lys Val Val
    130                 135                 140

Lys Pro Phe Gly Asp Ser Arg Met Tyr Asn Ile Thr Phe Thr Lys Val
145                 150                 155                 160

Leu Ser Val Cys Val Trp Val Ile Met Ala Val Leu Ser Leu Pro Asn
                165                 170                 175

Ile Ile Leu Thr Asn Gly Gln Pro Thr Glu Asp Asn Ile His Asp Cys
            180                 185                 190

Ser Lys Leu Lys Ser Pro Leu Gly Val Lys Trp His Thr Ala Val Thr
        195                 200                 205

Tyr Val Asn Ser Cys Leu Phe Val Ala Val Leu Val Ile Leu Ile Gly
    210                 215                 220

Cys Tyr Ile Ala Ile Ser Arg Tyr Ile His Lys Ser Ser Arg Gln Phe
225                 230                 235                 240

Ile Ser Gln Ser Ser Arg Lys Arg Lys His Asn Gln Ser Ile Arg Val
                245                 250                 255

Val Val Ala Val Phe Phe Thr Cys Phe Leu Pro Tyr His Leu Cys Arg
            260                 265                 270

Ile Pro Phe Thr Phe Ser His Leu Asp Arg Leu Leu Asp Glu Ser Ala
        275                 280                 285

Gln Lys Ile Leu Tyr Tyr Cys Lys Glu Ile Thr Leu Phe Leu Ser Ala
    290                 295                 300

Cys Asn Val Cys Leu Asp Pro Ile Ile Tyr Phe Phe Met Cys Arg Ser
305                 310                 315                 320

Phe Ser Arg Arg Leu Phe Lys Lys Ser Asn Ile Arg Thr Arg Ser Glu
                325                 330                 335

Ser Ile Arg Ser Leu Gln Ser Val Arg Arg Ser Glu Val Arg Ile Tyr
            340                 345                 350

Tyr Asp Tyr Thr Asp Val
        355

<210>  3
<211>  21
<212>  DNA
<213>  Artificial sequence

<220>
<223>  Target sequence for siRNA

<400>  3
gatcggaagt tcgcatatat t                                                21